# NEW EUROPEAN PATENT SPECIFICATION

(11) **EP 1 053 023 B2**
(45) Date of publication and mention of the opposition decision: **30.03.2011**
(45) Mention of the grant of the patent: 04.07.2007
(21) Application number: 99903557.9
(22) Date of filing: 03.02.1999
(51) Int. Cl.: A61K 48/00

(54) **SYSTEMIC DELIVERY OF SERUM STABLE PLASMID LIPID PARTICLES FOR CANCER THERAPY**
SYSTEMISCHE VERABREICHUNG VON SERUM-STABILEN PLASMID-LIPID PARTIKELN ZUR KREBSTHERAPIE
ADMINISTRATION SYSTEMIQUE DE PARTICULES LIPIDIQUES DU PLASMIDE STABLES DANS LE SERUM EN CANCEROTHERAPIE

(30) Priority: 03.02.1998 US 73598 P; 27.05.1998 US 86917 P; 22.09.1998 US 101429 P; 14.12.1998 US 112384 P; 02.02.1999 US 243102
(43) Date of publication of application: 22.11.2000
(73) Proprietor: Protiva Biotherapeutics Inc., Burnaby, British Columbia V5J 5J8 (CA)
(72) Inventor: MACLACHLAN, Ian, Vancouver, British Columbia V6J 1S8 (CA); GRAHAM, Roger, Vancouver, British Columbia V6K 1Z1 (CA)
(74) Representative: Campbell, Patrick John Henry
(86) International application number: PCT/CA1999/000090
(87) International publication number: WO 1999/039741

(56) References cited:
- WO-A-91/06309
- WO-A-95/05853
- WO-A-96/39831
- WO-A-96/40964
- NABEL, GARY J. (1) ET AL: "Direct gene transfer with DNA - liposome complexes in melanoma: Expression, biologic activity, and lack of toxicity in humans." PROCEEDINGS OF THE NATIONAL ACADEMY OF SCIENCES OF THE UNITED STATES OF AMERICA, (1993) VOL. 90, NO. 23, PP. 11307-11311. ISSN: 0027-8424., XP002110798
- MOOLTEN: "TUMOR CHEMOSENSITIVITY CONFERRED BY INSERTED HERPES THYMIDINE KINASE GENES: PARADIGM FOR A PROSPECTIVE CANCER CONTROL STRATEGY" CANCER RESEARCH, vol. 46, 1986, pages 5276-5281, XP000610741 cited in the application

## Description

### FIELD OF THE INVENTION

This invention relates to treating a metastatic neoplasia in a mammal.

### BACKGROUND OF THE INVENTION

Systemic delivery of therapeutic nucleic acids for gene therapy applications is a highly desirable goal. Systemic delivery using blood or lymph circulatory systems will permit therapeutic nucleic acids to seek out multiple distal disease sites, and to approach a disease site from multiple points. Successful systems will be useful against cancers (*i.e*., solid, nonsolid or metastatic tumors), infectious diseases, inflammation and other disease targets that are distal to the site of administration.

Currently, the most popular working systems for *in vivo* human gene therapy are not systemic delivery systems. Current systems employ direct, *i.e*., local, injection or inhalation of modified adenoviruses (*see*, Englehardt, "Methods for Adenovirus-Mediated Gene Transfer to Airway Epithelium," Chapter 11 in Methods in Molecular Medicine, Gene Therapy Protocols. Ed. P. Robbins, 1997. Humana Press Inc., Totowa, NJ), retroviruses ( Olsen, et al., "Methods for the Use of Retroviral Vectors for Transfer of the CFTR Gene to Airway Epithelium," Chapter 10, Methods in Molecular Medicine, *supra*), cationic lipid-plasmid aggregates (Nabel, et al., "Methods for Liposome-Mediated Gene Transfer to Tumor Cells in vivo," Chapter 21, Methods in Molecular Medicine, *supra;* Son, et al., "Cationic Liposome-Mediated Gene Transfer to Tumor Cells in Vitro and In vivo," Chapter 23, Methods in Molecular Medicine, *supra*), or simply delivery of naked DNA (*see,* U.S. Patent No. 5,589,466 to Felgner, et al.).

Unfortunately, there are well-known drawbacks to these popular methods that prevent their use as systemic delivery systems. For instance, cationic lipid-plasmid aggregates are rapidly cleared by the liver, lung or spleen after intravenous delivery and, therefore, do not satisfy the criteria for systemic delivery since they demonstrate little useful transfection elsewhere. This may be a result of their large diameter, *i.e.,* >200 nm, and powerful cationic surface charge. Moreover, adenoviral and retroviral systems are immunogenic, are rapidly cleared from circulation and do not permit repeat dosing. In addition, naked DNA is rapidly degraded in the blood and, thus, is not suitable for systemic delivery.

Systemic delivery for *in vivo* gene therapy, *i.e*., delivery of a therapeutic nucleic acid to a distal target cell via body systems such as the circulation, a less well explored avenue, has been achieved using lipid-plasmid particles such as those disclosed in published PCT Patent Application WO 96/40964, U.S. Patent No. 5,705,385, and U.S. Patent Applications Serial Nos. 08/485,458, 08/484,282, 08/660,025, 08/856,374, 60/073,598 and 60/063,473, all of which are assigned to the assignee of the instant invention. This latter format provides a fully encapsulated lipid-plasmid particle that protects the therapeutic nucleic acid from nuclease degradation in serum, is nonimmunogenic, is small in size and is suitable for repeat dosing.

Once the systemic delivery system has been established, the next question is to determine which disease condition to treat and which therapeutic nucleic acid to deliver. To date, no publication has reported therapeutic data employing, in a systemic delivery system, the variation of the gene therapy technique known as gene-delivered enzyme prodrug therapy ("GDEPT") or, alternatively, the "suicide gene/prodrug" system, which was first developed by Moolten, F.L., Cancer Res., 46:5276-5281 (1986). For a review of the GDEPT system, *see* Moolten, F.L., The Internet Book of Gene Therapy, Cancer Therapeutics, Chapter 11 (Sobol, R.E., Scanlon, NJ (Eds) Appelton & Lange (1995)). In this method, a heterologous gene is delivered to a cell, the heterologous gene encoding an enzyme that promotes the metabolism of a first compound to which the cell is less sensitive (*i.e.,* the "prodrug") into a second compound to which is cell is more sensitive. The prodrug is delivered to the cell either with the gene or after delivery of the gene. The enzyme will process the prodrug into the second compound and respond accordingly. A suitable system proposed by Moolten is the herpes simplex virus - thymidine kinase (HSV-TK) gene and the prodrug ganciclovir. This method has recently been employed using cationic lipid-nucleic aggregates for local delivery (*i.e*., direct intra-tumoral injection), or regional delivery (*i.e*., intra-peritoneal) of the TK gene to mouse tumors by Zerrouqui, et al., Can. Gen. Therapy, 3(6):385-392 (1996); Sugaya, et al., Hum. Gen. Ther., 7:223-230 (1996) and Aoki, et al., Hum. Gen. Ther., 8:1105-1113 (1997). Human clinical trials using a GDEPT system employing viral vectors have been proposed (*see,* Hum. Gene Ther., 8:597-613 (1997), and Hum. Gene Ther., 7:255-267 (1996)) and are underway.

Patent applications relating to the GDEPT method have been published under the following numbers: WO 97/19180; WO 97/07118; WO 96/22277; WO 97/19183; WO 96/16179; WO 96/03515; WO 96/03515; WO 96/03151; EP 690129; EP 657541; EP 657539; WO 95/05835 and EP 415731.

From the foregoing, it is readily apparent that systemic delivery of therapeutic nucleic acids to distal disease sites would clearly provide significant advantages over existing gene therapy modalities. It is an object of this invention to achieve this goal.

### SUMMARY OF THE INVENTION

The present invention provides, *inter alia,* a use according to claim 1.

The present invention can be used for sensitizing a neoplastic cell by a use according to claim 29.

The nucleic acid and the first compound can be delivered in lipid formulations that can be the same or different. The lipid formulations, whether used to deliver the nucleic acid or first compound (*e.g.,* prodrug), can be prepared from a variety of lipids, lipid conjugates and additional compatible components known in the art. The lipid formulations can be prepared, for example, from sphingomyelin and cholesterol. Moreover, the lipid formulations can contain additional components that improve the properties or characteristics of the formulations, such as leakiness, longevity in circulation, reduced toxicity, encapsulation efficiency, *etc*. Such components include, for example, cationic lipids, ATTA-lipid conjugates, PEG-lipid conjugates, targeting agents, *etc.*

Any therapeutic nucleic acid useful in treating metastatic neoplasia in a mammal can be administered. For instance, when the GDEPT system is employed, the nucleic acid can be any nucleic acid that encodes a gene product that promotes the processing, *i.e.,* conversion, of a first compound (*e.g.*, a prodrug) into a second compound to which the mammal or cell of interest is more sensitive or receptive. Examples of suitable gene-products include, but are not limited to, herpes simplex virus thymidine kinase, cytosine deaminase, xanthine-guaninephosphoribosyl transferase, purine nucleoside phosphorylase, cytochrome P450 2B 1 and their analogs. Other gene-products suitable for use in the methods of the present invention will be readily apparent to those of skill in the art.

In a preferred embodiment, the first compound is a prodrug, *i.e.,* a compound to which the cell of interest in not initially sensitive to, but which the gene-product converts into a compound to which the cell of interest is more sensitive. Examples of suitable prodrugs include, but are not limited to, ganciclovir, acyclovir, bromovinyldeoxyuridine, 5-fluorocytosine, 6-thioxanthine, MeP-dr and cyclophosphamide. Other prodrugs suitable for use in the methods of the present invention will be readily apparent to those of skill in the art.

Other features, objects and advantages of the invention and its preferred embodiments will become apparent from the detailed description which follows.

### BRIEF DESCRIPTION OF THE DRAWINGS

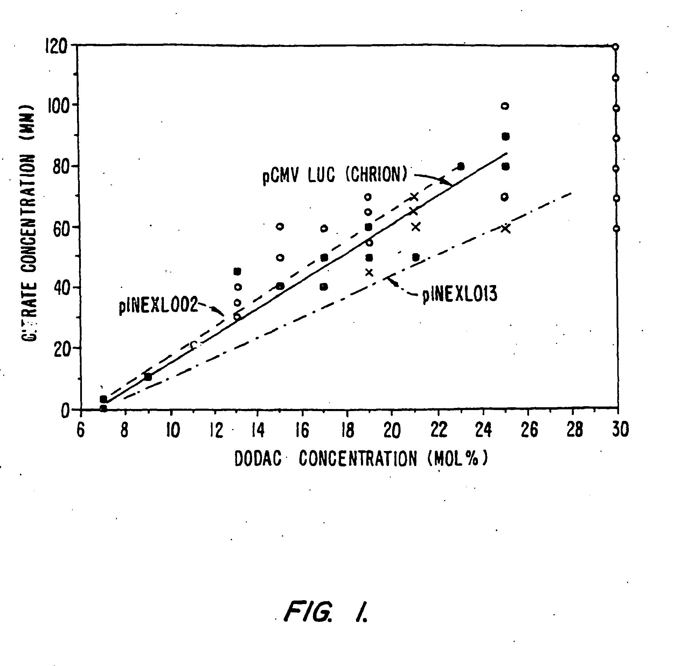

**Figure 1** illustrates the relationship of citrate concentration in the dialysis buffer and the DODAC mol% in the lipid for the preparation of lipid-plasmid particles. The solid dots represent good quality formulations having high association efficiencies (>40%), small size (<100 nm) and low values of size polydispersity (chi-square less than 10, preferably less than 3) on a NICOMP particle sizer. The stars represent formulations containing aggregates having large polydispersity values; and the open circles represent formulations having low association efficiencies (<40%). Proper tuning of the citrate buffer concentration to the cationic lipid charge appears to improve the formulation. Alternative anionic buffers can also be used if the counterions can prevent the cationic lipid from aggregating during the detergent removal step.
**Figure 2** illustrates the biodistribution of 303i in various organs (*i.e*., blood, spleen and liver) in C57-Lewis Lung mice.
**Figure 3** illustrates the accumulation of 303i at the tumor site in C57-Lewis Lung mice.
**Figure 4** illustrates a time course of gene product activity at distal (metastatic) tumor sites.
**Figure 5** illustrates gene expression in LS 180 tumors (dose response of 303i after 48 hours).
**Figures 6(A) and 6(B)** illustrate the pattern of HSV-TK gene expression within peritoneal tumors.
**Figure 7** illustrates the pINEX-TK10 construct which consists of a pBR322 derived plasmid containing a CMV promoter linked to a "hyper" HSV-TK gene, bovine growth hormone polyadenylation sequence and kanamycin resistance gene.
**Figure 8(A)** illustrates *in vivo* efficacy studies using a tumor model.
**Figure 8(B)** illustrates a 16-day treatment regimen on test mice after tumor inoculation.
**Figure 9(A)** illustrates the *in vivo* gene expression protocol.
**Figure 9(B)** illustrates an assessment of the tumor growth, with the empty formulation showing the largest tumor volume.
**Figure 9(C)** illustrates the efficacy of the suicide gene SPLP of this invention.
**Figure 10** illustrates the long-term survival following treatment with the suicide gene SPLP *(i.e.,* Formulation 1.1 or, alternatively, INEX 303 or 303i) of this invention.
**Figure 11** illustrates the efficacy of systemic delivery of TK303 in the BALB/c CT26 tumor model, *i.e.,* a colorectal tumor model.
**Figure 12** illustrates the effect of the TK FS (frame shift) construct, which does not express active TK polypeptide, and the TK construct on tumor volume.

### DEFINITIONS

"Sensitizing," as used herein, refers to the ability to increase the sensitivity of a designated system, such as a cell. This term includes changing a cell to make it responsive or more responsive to a compound to which it previously was not responsive, sensitive or was less sensitive. Sensitizing and "more sensitive" also includes changes to a cell such that exposure to a previously nonkilling substance results in cell death.

"Nucleic acid vector" or "vector," as used herein, refers to a composition comprising a nucleic acid sequence encoding a gene product. This is usually a plasmid or viral genome, but can also include other compositions, such as linear nucleic acids, protein/nucleic acid conjugates, *etc*. Depending on the use, vector can also refer to a nucleic acid delivered in a virus encapsulated or protein coated format, wherein the entire composition is known as a vector.

"Neoplasia," as used herein, refers to any aberrant growth of cells, tumors, malignant effusions, warts, polyps, nonsolid tumors, cysts and other growths. A site of neoplasia can contain a variety of cell types, including neoplastic cells, which harbor deleterious genetic mutations, normal cells which are induced by neoplasia, such as vascular endothelia, or immune system cells, such as macrophages and leukocytes, *etc*.

"Therapeutically effective amount," as used herein, refers to an amount that is sufficient or necessary to give rise to a desired therapeutic effect. The therapeutic effect can be obtained directly or indirectly. For instance, the therapeutic agent can lead to activation of other therapeutic agents or can act in combination with additional therapeutic agents. For neoplasia, a therapeutic effect can be, for example, a reduction in growth, inhibition or reduction in size of the neoplasia or inhibition or reduction of metastasis and other malignant attributes, or other beneficial effects, such as subjective or objective observations of physicians and patients.

"Gene product," as used herein, refers to a product of a gene such as an RNA transcript. The RNA transcript can be therapeutic of its own accord as in the case of antisense or ribozyme transcription plasmids, or the RNA transcript can be translated into a polypeptide that is also a gene product.

"Distal site," as used herein, refers to a physically separated site, which is not limited to an adjacent capillary bed, but includes sites broadly distributed throughout an organism.

"Serum-stable" in relation to lipid/therapeutic nucleic acid particles means that the particle is not significantly degraded after exposure to a serum or nuclease assay that would significantly degrade free DNA. Suitable assays include, for example, a standard serum assay or a DNAse assay such as those described in the Example section.

"Systemic delivery," as used herein, refers to delivery that leads to a broad biodistribution of a compound within an organism. Some techniques of administration can lead to the systemic delivery of certain compounds, but not others. Systemic delivery means that a useful, preferably therapeutic, amount of a compound is exposed to most parts of the body. To obtain broad biodistribution generally requires a blood lifetime such that the compound is not rapidly degraded or cleared (such as by first pass organs (liver, lung, *etc.*) or by rapid, nonspecific cell binding) before reaching a disease site distal to the site of administration. Systemic delivery of lipid/therapeutic nucleic acid particles is preferably obtained by intravenous delivery.

### DETAILED DESCRIPTION OF THE INVENTION AND PREFERRED EMBODIMENTS

The present invention provides for treating a metastatic neoplasia with a therapeutic nucleic acid, preferably a plasmid encoding an expressible gene, wherein the therapeutic nucleic acid is encapsulated in a lipid particle and administered intravenously at a site distal to the site of a metastatic neoplasia and delivered to the site of neoplasia by systemic delivery.

The invention takes advantage of lipid-nucleic acid particles, wherein the nucleic acid is fully encapsulated and protected from nuclease degradation, and wherein the particles have a small diameter (50-200 nm) and have other attributes suitable for systemic delivery.

In general, patient therapy using the invention is achieved as follows. First, the particles are prepared using lipids and a therapeutic nucleic acid. Thereafter, the particles are administered to a mammal having a metastatic neoplasia by intravenous injection at a site that is distal to the site of neoplasia. The particles are delivered to the site of neoplasia by the blood, lymph or other internal body fluid. A therapeutically effective amount of particles accumulates at the site of neoplasia, and are taken up by cells at the site. Therapeutic effects are obtained at the site of neoplasia generally by transcription of the nucleic acid that leads either to a therapeutic transcription product *(i.e.,* a ribozyme or antisense oligonucleotide or other RNA), or to an mRNA that is translated into a therapeutic polypeptide.

The benefits of systemic gene therapy against tumors using lipid/therapeutic nucleic acid particles include, but are not limited to, the following benefits.

First, nucleic acids are typically nontoxic in comparison to other chemotherapeutic agents employed. Second, the lipid carrier systems of the present invention demonstrate preferential accumulation at many tumor sites. This phenomena depends, in part, on the "leakiness" of the neo-vasculature of the tumor. This preferential "passive" targeting can be enhanced by targeting agents attached to the outer lipid monolayer, such as FGF which enhances localization of lipid particles at the tumor site (*see, e.g.,* Forum, et al., "Liposome Targeting in Animal Models," L. Huang (Ed.), Journal of Liposome Research, 7(4):315-534 (1997)). Third, therapeutic nucleic acids potentially provide more efficient therapeutics than convention drugs because they are capable of replication at the site of disease. The many copies of transcripts or gene products generated reduce the total number of active agents that must be delivered to the cells. Further, therapeutic nucleic acids can generate polypeptides within the target cells, which could otherwise be too expensive to manufacture or too difficult to deliver. Fourth, therapeutic nucleic acids, by leading to synthesis of polypeptides within the disease cell, can be used to deliver polypeptides to sites inside the disease cell to which the polypeptide could not be delivered from exogenous routes. Finally, because of their low toxicity, therapeutic nucleic acids can be suitable candidates for combination therapy with other chemotherapeutic agents. A suitable combination therapy approach is described in U.S. Provisional Patent Application Serial No. 60/082,665 (TTC Attorney Docket No. 16303-007100), which is assigned to the assignee of the instant invention.

To date, several approaches for introducing nucleic acids into cells *in vivo* have been used. These include liposome based gene delivery, intratracheal instillation, aerosolized gene delivery or direct injection. For example, Debs and Zhu WO 93/12240, Debs WO 92/1108 and Debs U.S. Patent No. 5,641,662 all describe aerosolized gene delivery of lipid DNA complexes to mammals. Similarly, Stribling, et al., PNAS, 89:11277-11281 (1992), describe lipid delivery to mice. McLachlan, et al., Gene Therapy, 2:614-622 (1995), describe DOTAP-mediated lipid delivery of hCFTR to mice. Canonico, et al., Am. J. Respir. Cell Mol. Biol., 10:24-29 (1994), and Canonico, et al., The American Physiological Society, 415-419 (1994), describe lipofectin-mediated gene delivery of há1AT to rabbits by aerosolized gene delivery. Alton, et al., Nature Genetics, 5:135-142 (1993), describe DC-chol:DOPE/ DOTAP-mediated delivery of hCFTR and â-gal by aerosol or tracheal instillation to mice. Capelen, et al., Nature Medicine, 1(1):39 (1995), describe delivery of CFTR to the nasal epithelia of Humans using a DC-Chol/DOPE mediated procedure, as does McLachlan, et al., Gene Ther., 3(12):1113-23 (1996). A variety of reports of administration of lipid-DNA complexes by parenteral administration have also been made, including Brigham WO 91/06309, U.S. Patent No. 5,676,954, and Debs and Zhu WO 93/24640. Accordingly, a variety of procedures for transducing cells *in vivo* using lipid-mediated techniques are known. However, such procedures have not been shown to be useful for systemic delivery. Details of preferred formulations of the present invention are given below.

### A. Therapeutic Nucleic Acids

The compositions and methods of the present invention are useful for delivering a wide variety of therapeutic nucleic acids. These nucleic acids can encode therapeutic polypeptides (*i.e*., any therapeutic gene product), or therapeutic polynucleotides (*i.e*., antisense or ribozyme transcription plasmids). The therapeutic nucleic acids of the invention can be expressible genes, such as those just described, or they can be nucleic acids, which by themselves, induce some form of response, perhaps by immune system stimulation.

For use with the instant invention, the most preferred therapeutic nucleic acids are those which are useful in gene delivered enzyme prodrug therapy ("GDEPT"). Any suicide gene/prodrug combination can be used in accordance with the present invention. Several suicide gene/prodrug combinations suitable for use in the present invention are cited in Sikora, K. in OECD Documents, Gene Delivery Systems at pp.59-71 (1996) include, but are not limited to, the following:

| **Suicide Gene Product** | **Less Active ProDrug** | **Activated Drug** |
|---|---|---|
| Herpes simplex virus type | ganciclovir(GCV), | phosphorylated dGTP |
| 1 thymidine kinase (HSV- | acyclovir, bromovinyl- | analogs |
| TK) | deoxyuridine, or other | |
| | substrates | |
| Cytosine Deaminase (CD) | 5-fluorocytosine | 5-fluorouracil |
| Xanthine-guanine- | 6-thioxanthine (6TX) | 6-thioguano- |
| phosphoribosyl transferase | | sinemonophosphate |
| (XGPRT) | | |
| Purine nucleoside | MeP-dr | 6-methylpurine |
| phosphorylase | | |
| Cytochrome P450 2B1 | cyclophosphamide | [cytotoxic metabolites] |
| Linamarase | amygdalin | cyanide |
| Nitroreductase | CB 1954 | nitrobenzamidine |
| Beta-lactamase | PD | PD mustard |
| Beta-glucuronidase | adria-glu | adriamycin |
| Carboxypeptidase | MTX-alanine | MTX |
| Glucose oxidase | glucose | peroxide |
| Penicillin amidase | adria-PA | adriamycin |
| Superoxide dismutase | XRT | DNA damaging agent |
| Ribonuclease | RNA | cleavage products |

Any prodrug can be used if it is metabolized by the heterologous gene product into a compound to which the cell is more sensitive. Preferably, cells are at least 10-fold more sensitive to the metabolite than the prodrug.

Modifications of the GDEPT system that may be useful with the invention include, for example, the use of a modified TK enzyme construct, wherein the TK gene has been mutated to cause more rapid conversion of prodrug to drug *(see,* for example, Black, et al., PNAS (USA), 93:3525-3529 (1996)). Alternatively, the TK gene can be delivered in a bicistronic construct with another gene that enhances its effect. For example, to enhance the "bystander effect" also known as the "neighbor effect" (wherein cells in the vicinity of the transfected cell are also killed), the TK gene can be delivered with a gene for a gap junction protein, such as connexin 43. The connexin protein allows diffusion of toxic products of the TK enzyme from one cell into another. The TK/Connexin 43 construct has a CMV promoter operably linked to a TK gene by an internal ribosome entry sequence and a Connexin 43-encoding nucleic acid.

In the second step, the prodrug is delivered to the cells. The prodrug can be the free drug or, alternatively, it can be in a lipid formulation. The use of lipid formulations in the GDEPT system has many surprising and previously undiscovered advantages over the delivery of free drug including, but not limited to, improved targeting to the disease site transfected by the vector, prolonged circulation half-life, increased drug loading, reduced toxicity towards nontarget tissues, improved treatment modalities, such as a single bolus injection as opposed to IV drip, and the like. These advantages overcome the limitations of the previously-known GDEPT systems. Further, the liposomal formulation of the prodrug will preferably provide similar biodistribution to a lipid vector formulation, thereby concentrating both the vector and the prodrug at the disease site.

Usually, the vector will be delivered to the target cell in advance of the prodrug in order to allow synthesis of the suicide gene product prior to the arrival of the prodrug. Temporal separation can be obtained either by separate administration of vector and prodrug or, alternatively, by providing the formulations simultaneously, wherein the vector formulation rapidly accumulates at the target site and delivers the vector, and the prodrug formulation accumulates or delivers its payload more slowly. As such, using the compositions and methods of the invention, the vector is delivered to the cell to direct synthesis of the suicide gene product, the cell is thereby sensitized, the prodrug is delivered to the cell, and patient therapy, reduction of tumor size is achieved.

In addition to the GDEPT systems, there exists a very wide variety of therapeutic nucleic acids that can be employed in the instant invention. The nucleic acids can be human, nonhuman (*i.e.*, from any other plant, animal or microorganism) or entirely synthetic (*i.e*., non-naturally occurring). The nucleic acids can be endogenous to the cells of the patient, or can be exogenous, meaning that the nucleic acid is not normally found in cells of the patients. Since treatment of neoplasia does not necessarily require long term or stable expression of the delivered nucleic acid, genes effective in transient expression systems, such as toxins or immune stimulatory proteins, are also useful in the methods of the present invention.

When the therapeutic nucleic acid is one that is endogenous to the patient, a modified sequence, an increased copy number, or a construct that has increased transcriptional activity relative to the native gene can be delivered. The gene product can be directly toxic, indirectly toxic or it can induce apoptosis or cell differentiation. In the most preferred system, the gene product of the therapeutic gene will demonstrate low toxicity to nontarget tissues, and high toxicity to the disease site. For example, when delivered in the preferred lipid-nucleic acid particles of the invention, the gene product preferably has greater toxicity to tumor cells than liver or spleen cells, where a large portion of particles are normally cleared. Disease site specificity can also be enhanced by employing tissue/disease specific promoters for gene transcription or translation. Tissue specific promoters, and methods of associating them with therapeutic nucleic acids are known to those skilled in the art.

Preferred endogenous genes suitable for use in the methods of this invention include, but are not limited to, pro-apoptotic genes; poreifirin; tumor suppressor genes (p53 and the like); cytokines (IL-2, IL-12, IL-15, GM-CSF, *etc.*); heat shock proteins; immunodominant Ag (or tumor-specific protein genes); genes activated in embryos only; TIMP-2 (tissue inhibitor of metallo proteinase-2) and other metastasis inhibiting proteins; and anti-angiogenic genes, such as endostatin or angiostatin *(see,* WO 97/15666; WO 95/29242; Boehm, et al., Nature, 309:404-407 (1997); and Kerbel, et al., Nature, 309:335 (1997)). IL-12 is a preferred endogenous gene that can be employed as a therapeutic nucleic acid in the instant invention *(see,* Tahara, H. and Lotze, M.T., Gene Ther., 2:96-106 (1995)). A suitable IL-12 plasmid construct for delivery is pNGVL3-mIL12 provided by the National Gene Therapy Vector Laboratory at the University of Michigan (Ann Arbor, Michigan).

Exogenous genes which are not naturally found in the cells of the patients, can be advantageous because their gene products can also serve to induce an immune response. For example, genes used in a suicide gene/pro-drug system can have this effect.

Preferred exogenous genes include, but are not limited to, genes used in GDEPT combinations (treatment in conjunction with pro-drugs); ribozymes or transcription plasmids encoding ribozymes or antisense transcripts; toxin genes, such as saporin, ricin, diphtheria toxin and cholera toxin (or any other plant, bacterial or fungal gene); viral protein genes, such as E1A; mutated E6; SV40 Tag, *etc.* Other exogenous genes suitable for use in the methods of the present invention will be readily apparent to those of skill in the art.

Methods of constructing plasmids or other vectors that carry the therapeutic nucleic acids disclosed herein are well known to those skilled in the art *(see, e.g.,* CURRENT PROTOCOLS IN MOLECULAR BIOLOGY (Ausubel, et al. (eds.) 1995). Therapeutic activity can be enhanced by the addition of transcription or translation promoters and other nucleic acid elements, again, all of which are known to those skilled in the art.

Lipid-nucleic acid and the lipid-prodrug formulations can be achieved by any known method. The preferred methods result in a high efficiency encapsulation, thereby reducing wastage and expense of the formulation. Lipid-nucleic acid and the lipid-prodrug formulations can be synthesized using standard freeze-thaw and extrusion techniques disclosed by Hope, et al., Biochim. Biophys. Acta, 812:55-65 (1985). In addition, other drug loading and encapsulation techniques that can be used are disclosed in U.S. Patent Application Serial Nos. 08/399,692, 08/607,614, 08/588,542, 08/741,622. Sizing of the lipid formulations can be achieved using extruders, pressure cells, and other tools known to those of skill in the art.

The possible lipid components of the lipid-nucleic acid and lipid-prodrug formulations of the invention include those components typically used in the art. For instance, sphingosomes, which are disclosed in U.S. Patent No. 5,543,152, and U.S. Patent Applications Serial Nos. 08/536,584, 08/316,399, 08/485,608, 08/442,267, can be used in the formulations.

### B. Preparing the Lipid/Therapeutic Nucleic Acid Particle

The methods for preparing lipid-nucleic acid formulations for systemic delivery result in a high-efficiency encapsulation, wherein little of the nucleic acid is exposed to free solution or adsorbed to the outer surface of the lipid particle. Such methods are disclosed in published PCT Patent Application WO 96/40964, U.S. Patent No. 5,705,385, and U.S. Patent Applications Serial Nos. 08/485,458, 08/484,282, 08/660,025, 08/856,374, 60/073,598 and 60/063,473, all of which are assigned to the assignee of the instant invention. Generally, high efficiency encapsulation provides low immunogenicity and improved tolerance when injected for systemic delivery. Further, these lipid-nucleic acid particles are relatively easy to characterize and define compared to cationic lipid-plasmid aggregates used in local delivery methods.

Preferred encapsulation methods are set out in the Example section. The lipid-therapeutic nucleic acid particles obtained by these methods have identifiable characteristics which make them suitable for use in the invention. For instance, they are small particles typically having a mean particle size of about 50 to about 200 nm and, preferably, of about 60 to about 130 nm. Most preferably, particles are of a relatively uniform size and have a χ² (chi-squared) value of less than about 3, more preferably of less than about 1 and, more preferably, of less than about 0.5.

Moreover, the lipid-therapeutic nucleic acid particles of the present invention are serum-stable and, thus, not significantly degraded after exposure to a serum or nuclease assay that would significantly degrade free DNA. Suitable assays for measuring serum stability include a standard serum assay or a DNase assay (which are described in the Example section). Nuclease resistance/serum stability is a measure of the ability of the formulation to protect the therapeutic nucleic acid from nuclease digestion either in an *in vitro* assay or in circulation. The encapsulated particles of the present invention have greater nuclease resistance and serum stability than lipid-plasmid aggregates (also known as cationic complexes), such as DOTMA/DOPE (LIPOFECTIN^{™}) formulations.

In addition, the lipid-therapeutic nucleic acid particles of the present invention have a nucleic acid to lipid ratio that can be formulated at various levels. For use in the methods of this invention, the particles have a drug to lipid ratio of at least about 3 mg of nucleic acid per mmol of lipid, more preferably, at least about 14 mg of nucleic acid per mmol of lipid and, even more preferably, greater than about 25 mg of nucleic acid per mmol of lipid. The preferred particles, when prepared to an administration ready formulation, are about 60 - 80 mg nucleic acid per mmol lipid (*i.e.,* they are "high ratio" formulations). The method used for making high ratio formulations can also be employed using reduced amounts of DNA to obtain lower ratio formulations. As used herein, "drug to lipid ratio" refers to the amount of therapeutic nucleic acid (*i.e.,* the amount of nucleic acid that is encapsulated and that will not be rapidly degraded upon exposure to the blood) in a defined volume of preparation divided by the amount of lipid in the same volume. This may be determined on a mole per mole basis, on a weight per weight basis, or on a weight per mole basis. For final administration ready formulations, the drug to lipid ratio is calculated after dialysis, chromatography and/or nuclease digestion have been employed to remove as much of the externally associated therapeutic agent as possible. Drug to lipid ratio is a measure of potency of the formulation, although the highest possible drug to lipid ratio is not always the most potent formulation.

An alternative description of the lipid-nucleic acid particles of the present invention is "high efficiency" formulations that emphasizes the active loading process involved and contrasts with low efficiency or passive encapsulation. Passive encapsulation of nucleic acid in lipid particles, which is known in the art, achieves less than 15% encapsulation of therapeutic agent, and results in low ratio particles having less than 3 mg of nucleic acid per mmol of lipid. The preferred lipid/therapeutic nucleic acid particles of the present invention have an encapsulation efficiency of greater than about 30%. As used herein, "encapsulation efficiency" refers to absolute efficiency, *i.e*., the total amount of DNA added to the starting mixture that ends up in the administration competent formulation. Sometimes the relative efficiency is calculated, wherein the drug to lipid ratio of the starting mixture is divided by the drug to lipid ratio of the final, administration competent formulation. The amount of lipid lost during the formulation process may be calculated. Efficiency is a measure of the wastage and expense of the formulation.

Other beneficial features that flow from the use of the preferred particles of the present invention, such as low nonspecific toxicity, improved biodistribution, therapeutic efficacy and ease of manufacturing, will be apparent to those of skill in the art. It is possible to develop particles as described above by alternative methods of encapsulation. These methods may employ standard techniques for loading of liposomes that are well known for use with conventional drugs. These methods include freeze-thaw extrusion, dehydration/rehydration, reverse phase evaporation, and the like, some of which are disclosed in Monnard, et al., "Entrapment of nucleic acids in liposomes, " Biochim. Biophys. Acta., 1329:39-50 (1997). These methods are not high encapsulation efficiency formulations, nor high ratio formulations, but the instant disclosure suggests the utility of such particles in the use of gene therapy against distal tumor sites.

In addition to the lipids employed in the methods used above, there are a tremendous number of additional lipid and nonlipid components which can be used to enhance delivery or targeting of particles. Additional lipid components include, but are not limited to, lipids with neutral, anionic, cationic or zwitterionic headgroups, and the like. These standard components are set out in the art and in the patent applications referred to above. Charged lipids that are particularly preferred with the invention are N,N-dioleyl-N,N-dimethylammonium chloride (DODAC), the subject of recently issued U.S. Patent No. 5,753,613, incorporated herein by reference and assigned to the assignee of the instant invention; and 1,2-Dioleoyl-3-dimethylammonium-propane (DODAP), the subject of U.S. Patent Application Serial No. 08/856,374.

Both the nucleic acid and prodrug formulations can include additional components selected from a wide variety of lipids, lipid conjugates and compatible additional components known in the art. For instance, cholesterol and its derivatives can be used in the nucleic acid and prodrug formulations of the present invention. Still other formulations can employ polycationic compounds that can condense DNA into small sizes before lipid encapsulation. Polylysine and polyethyleneimine, among other compounds, have been used by those of skill in the art in this capacity. Condensed particles can also be used in the methods of this invention.

In addition, cloaking agents can be used to reduce elimination by the host immune system. Such cloaking agents include, for example, polyamide oligomer-lipid conjugates, such as ATTA-lipids, disclosed in U.S. Patent Application Serial No. 08/996,783, filed February 2, 1998 (TTC Attorney Docket No. 16303-005800) and PEG-lipid conjugates disclosed in U.S. Patent Application Serial Nos. 08/486,214, 08/316,407 and 08/485,608. These components can also be targeting agents that encourage the lipid formulations to accumulate at the area of the disease or target site. In addition, these components can be compounds that improve features of the formulation, such as leakiness, longevity in circulation, reduction in toxicity, encapsulation efficiency, *etc*. Examples of these components and others that can usefully be included in the formulations of the invention are known to and used by those skilled in the art.

With respect to both the nucleic acid and prodrug lipid formulations, it is sometimes preferable to employ a programmable fusogenic lipid formulation. This refers to a formulation that has little tendency to fuse with cell membranes and deliver its payload until a given signal event occurs. This allows the lipid formulation to distribute more evenly after injection into an organism or disease site before it starts fusing with cells. The signal event can be, for example, a change in pH, temperature, ionic environment, or simply time. In this last event, a fusion delaying or "cloaking" component, such as an ATTA-lipid conjugate or a PEG-lipid conjugate, can simply exchange out of the liposome membrane over time. By the time the formulation is suitably distributed in the body, it is calculated to have lost sufficient cloaking agent so as to be fusogenic. With other signal events, it may be desirable to choose a signal event that is associated with the disease site or target cell, such as increased temperature at a site of inflammation.

One of the great advantages of the invention is its versatility in targeting a broad range of disease sites. In particular, lipid encapsulated formulations are usefully employed in targeting and killing metastatic neoplasia.

The nucleic acid, *e.g*., vector, is delivered in a lipid encapsulated formulation by intravenous administration. This mode of administration takes advantage of the known tendency of lipid encapsulated formulations to accumulate at tumors and neoplasia even without specific targeting aspects. This ability is thought to be the result of "leaky" vasculature at sites of neoplasia which is easily invaded by small sized lipid particles *(see,* Jain, Sci. Am., 271:58-65 (1994)).

Where specific cell type targeting is preferred, the lipid formulation can contain, on the outer surface, antigens or markers that are recognized by moieties or that recognize receptors on the target cell. Examples of such targeting can be found in, for example, Forum, et al., "Liposome Targeting in Animal Models," L. Huang (Ed.), Journal of Liposome Research, 7(4):315-534 (1997).

### C. Administration-Ready Pharmaceutical Preparations

Generally, when administered intravenously, the nucleic acid and/or the prodrug formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, PA, 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, *etc.* Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, *etc.* These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration. Carriers may also be employed when delivering the vector or prodrug formulations by other parenteral methods known in the art, such as subcutaneous, intratumoral or intramuscular injection, inhalation, and the like.

When preparing pharmaceutical preparations of the lipid/therapeutic nucleic acid particles of the invention, it is preferable to use quantities of the particles which have been purified to reduce or eliminate empty particles or particles with nucleic acid associated with the external surface.

### D. Disease Indications Suitable for Treatment by the Invention

The instant invention is useful for treatment of metastatic neoplasia in mammals. Treatment means obtaining a therapeutic effect at the site of neoplasia. Treatment of a diverse range of metastatic tumors can be obtained using the compositions of this invention. In addition, the compositions and methods of this invention can be tested against standard NIH-recommended models. *See,* for example, Driscoll, "The preclinical new drug research program of the National Cancer Institute," Cancer Treatment Reports, 68:63-76 (1984). Further, *in vivo* and/or in vitro models that are routinely employed in National Cancer Institute sponsored drug screening evaluations for identifying utility against human neoplasia can be employed to confirm the utility of the instant invention (*See,* Boyd, M.R., "The NCI In Vitro Anticancer Drug Discovery Screen. In Anticancer Drug Development Guide" (B. Teicher (ed.), 1995, Humana Press, Totawa, NJ).

Characteristics of metastatic neoplasia against which this invention is useful include, but are not limited to, tumors or neoplasia that are 1) reasonably transfectable by lipid/therapeutic nucleic acid particles; 2) are responsive to the gene product of the nucleic acid; and 3) are not readily accessible to surgical approaches. Such tumors include micro-metastases, metastasis found outside of the lung, liver or spleen ("first pass organs"), and the like. Thus, the invention is applicable to a variety of tumor types. A key characteristic of a suitable disease indication will be its accessibility to the lipid/therapeutic nucleic acid particles of the invention.

### E. Combination Therapy

Combination chemotherapy, a well-known technique employing conventional drugs for treating cancer, can also be employed using the lipid-nucleic acid particles of the invention. Combination chemotherapy treatment has advantages in that: 1) it avoids single agent resistance; 2) in a heterogenous tumor population, it can kill cells by different mechanisms; and 3) by selecting drugs with nonoverlapping toxicities, each agent can be used at full dose. Combination regimens, which are curative when single agent treatment is not, include, but are not limited to, the following: acute lymphocytic leukemia - vincristine, prednisone, doxorubicin and L-asparaginase; Hodgkin's disease - mechoroethamine, vincristine, procarbazine and prednisone (MOPP); histiocytic lymphoma - cyclophosphamide, vincristine, procarbazine and prednisone (C-MOPP); and testicular carcinoma - bleomycin, vinblastine, cisplatin.

Some considerations that are well known in selecting drug combinations include the following:
Kinetic considerations: A heterogenous tumor will be treated first with noncell cycle specific agents (e.g., cyclophosphamide and doxorubicin) to debulk the tumor and recruit slowly dividing cells into active DNA synthesis; followed by a cell cycle specific drug, such as methotrexate and 5-FU. The drug cycle is repeated at regular intervals.
Drug resistance considerations: Two or more non-cross resistant drugs are used simultaneously to avoid selection of resistant tumor cells. Double resistant mutants may arise by sequential chemotherapy.
Drug interactions: Some combinations *(i.e.,* methotrexate and 5-FU) are synergistic when given in the proper sequence, antagonistic when the order is reversed. Some combinations (*i.e*., L-Asparaginase and methotrexate) are antagonistic initially, but after an extended period (about 10 days), tumor cells are found to be more sensitive to methotrexate.

All these considerations may play a role in the proper selection of therapeutic genes and anti-neoplastic agents employed. Some possible combination therapies employing gene therapy and chemotherapeutic agents are set out in U.S. Patent Application Serial No. 60/082,665 (TTC Attorney Docket No. 16303-007100), assigned to the assignee of the instant invention. It is particularly useful to deliver therapeutic nucleic acids to metastatic neoplasia which have been identified as multi-drug resistant, in order to resensitize the tumor to the chemotherapeutic agent.

### F. Dosages of Drugs

The precise dosage to be administered to a patient, whether as part of the GDEPT system or as part of combination chemotherapy, will ultimately be dependant upon the discretion and professional judgement of the attendant physician and will be in part dependent on such factors as the age, weight and the particular metastatic neoplasia of the patient. The amounts and precise regime will of course depend on other factors including the severity of the condition to be treated.

In other systems, the exact dosage regime will need to be determined by individual clinicians which will be controlled by the exact nature of the nucleic acid to be delivered and the condition to be treated, but some general guidance can be given. In general, dosage can easily range from about 0.1 µg to 1 g or more of nucleic acid. More preferably, the dose of nucleic acid will range from about 0.1 µg to about 5 mg per kilogram for a typical 70 kilogram patient, and doses of vectors, which include a viral particle, are calculated to yield an equivalent amount of therapeutic nucleic acid.

In GDEPT systems, a suitable dose of the nucleic-acid lipid particle will be the amount of nucleic acid which will produce about 500 to about 200,000 enzyme units/m² (*e.g*., 20,000 enzyme units/m²). The dose of the prodrug will advantageously be in the range of about 0.1 to 250 mg per kilogram of body weight of recipient per day, preferably about 0.1 to 100 mg per kilogram bodyweight.

In combination chemotherapy, a suitable dose of the nucleic acid will typically range from about 0.1 µg to about 5 mg per kilogram for a typical 70 kilogram patient. In addition, a suitable dose of the other drug, *e.g*., the other anti-cancer agent, will advantageously be in the range of about 0.1 to 250 mg per kilogram of body weight of recipient per day and, more preferably, in the range of about 0.1 to 100 mg per kilogram body weight of recipient per day.

Typically, the particle will be administered to the patient and then the uptake and transfection into cells will be monitored, for example by recovery and analysis of a biopsy sample of the targeted neoplastic tissue. This can be determined by clinical trials which involve administering a range of trial dosages to a patient and measuring the degree of transfection in a target cell or tumor. In the methods of the current invention, the prodrug will usually be administered following administration of the nucleic acid encoding a gene product.

The invention will be described in greater detail by way of specific examples carried out in accordance with Canadian Council on Animal Care, 2nd Ed., "Guide to the care and use of experimental animals," Eds. Olfert, E., Cross, B. and McWilliam, A. (1993). The following examples are offered for illustrative purposes, and are not intended to limit the invention in any manner. Those of skill in the art will readily recognize a variety of noncritical parameters which can be changed or modified to yield essentially the same results.

### EXAMPLES

The examples which refer to neoplasias other than metastatic neoplasias are indicated for comparative purposes.

### EXAMPLE 1

This example illustrates the synthesis of 5 lipid-plasmid particle formulations for systemic delivery.

Materials: Plasmids are preferably supercoiled, 4000 to 15000 bp in length, encoding genes and enhancer elements, *etc.* as desired. Cationic lipid, N,N-dioleyl-N,N-dimethyl ammonium chloride ("DODAC") and monomethoxy polyethylene2000 glycol succinate-(C8:0-ceramide) ("PEG-Cer-C8") were synthesized at Inex Pharmaceuticals Corp. Dioleyl-phosphatidylethanolamine (DOPE) was supplied by Northern Lipids, Vancouver. Standard dialysis membranes: Spectro/Por 5 regenerated Cellulose (12-14,000 MWCO) was purchased from VWR (Manufactured by Spectrum Medical Industries Inc.). Sodium Citrate was purchased from BDH. Sodium Chloride, Triton X-100 and Octyl-beta-D-glucopyranoside ("OGP") were obtained from VWR Scientific, Fisher Scientific or Sigma Chemical Company.

### Formulation 1.1 (Or, Alternatively, INEX 303 or INEX 303i)

Plasmid (50-400 µg) is incubated with DODAC in 500 µL of the prep solution containing 0.2 M OGP in 150 mM NaCl; 5 mM HEPES pH 7.4, for 30 min at room temperature. This mixture is added to a mixture of DOPE and PEG-Cer-C14 or PEG-Cer-C20 or PEG-Cer-C8 in 500 µL of the same prep solution. The total lipid concentration was either 5 or 10 mg/mL, with the molar ratio of DOPE:DODAC:PEG-Cer being 84:6:10. The mixture was dialyzed against 150 mM NaCl; 5 mM HEPES (pH 7.4) for 36-48 h with two buffer changes.

Nonencapsulated DNA was removed by anion exchange chromatography on DEAE-Sepharose column (1X4 cm). Empty liposomes were removed by pooling lipid/DNA samples that co-eluted on the DEAE column on top of a sucrose density gradient in 12.5 mL ultracentrifuge tubes. The gradient was formed with 3 mL each of 10% sucrose, 2.5% sucrose and 1% sucrose in HBS layered consecutively from bottom to top. The gradients were centrifuged at 36,000 rpm (160,000 X g) for 2 h at 20°C in a Beckman Optima XL-100K ultracentrifuge using an SW-28 rotor. Separated bands were removed from top to bottom. Fractions were assayed for 3H-plasmid and 14C-CHE by dual-label scintillation counting using a Beckman LS6500 scintillation counter. The lipid encapsulated plasmid DNA banded tightly at the interface between 2.5% and 10% sucrose, while the unassociated lipid was present as a smear from the top of the gradient to the interface between 1% and 2.5% sucrose. The formulation can be concentrated in 12-14,000 MWCO dialysis tubing against 500,000 MW PEG (Aquacide II). When the desired volume is reached, the formulation was transferred into a new dialysis bag and dialyzed overnight against HBS to adjust the NaCl concentration to 150 mM.

### Formulation 1.2 (Or, Alternatively, INEX 351)

In formulation 1.2 the following concentrations were used. Lipid concentration: 5.0 mg/mL (or 5.3 mM); plasmid concentration, 200 ig; initial volume, 1.0 mL; lipid stock solutions (in 95:5 benzene:methanol, 2:1 chloroform:methanol or ethanol). Calculated by molarity (dissolved in 95:5 benzene:methanol or 2:1 chloroform:methanol). DOPE (744 g/mol), 40 mM; DODAC (582 g/mol), 40 mM; and PEG-C8 (2515 g/mol), 20 mM.

### Formulation for 351: 42.5:42.5:15 (mole %) DOPE:DODAC:PEG-C8

| | DOPE | DODAC | PEG-C8 |
|---|---|---|---|
| mg | 1.68 | 1.315 | 2.005 |
| mole % | 42.5 | 42.5 | 15 |
| µmol | 2.25 | 2.25 | 0.8 |
| µl | 56.2 | 56.2 | 40 |

### Formulation Procedure (1 ml scale):

Aliquot lipid stock solutions into a clean, dry test tube and dry to a lipid film using a stream of N₂ gas and then dry under vacuum for at least 2 hrs. Add 50 µL 2M OGP and add 500 µL of 2X strength dialysis buffer, add 200 µg of plasmid and mix by vortexing to dissolve the lipid film. Make up to 1.0 mL with sterile deionized H₂O, mix and allow to incubate approximately 30 min at room temperature. Place the solution into a dialysis bag and dialyze for 40-48 hrs against 2 L of dialysis buffer with 1-2 changes of buffer after approximately 24 hrs, and determine the volume of the sample by weighing in a tarred tube (assume density of 1.0). These steps may be followed by DEAE cleaning and/or sucrose density gradient centrifugation, as described above.

After DEAE cleaning and sucrose density centrifugation, as described above, the final INEX 351 formulation has a concentration of about 200 µg/ml plasmid and 5 mg/ml total lipid.

| NOTES for INEX 351: | |
|---|---|
| Note 1: | Appropriate dialysis buffer concentrations: p53 : 150 mM NaPO₄ + 150 mM NaCl (try 140 - 160 mM NaCl), pH 7.4 pLuc: + 175 mM NaCl (about 150 - 170 mM NaCl), pH 7.4 |
| Note 2: | 150 mM NaPO₄ buffer, pH 7.4: 35.77 g dibasic sodium phosphate (Na₂HPO₄) 6.62 g monobasic sodium phosphate (NaH₂PO₄) add appropriate quantity of NaCl dissolve in 2 L (final volume) of deionized water with stirring. The final pH may vary between a pH of about 7.3 and about 7.4; this has not normally been adjusted and has not affected the performance of the formulation. |
| Note 3: | Use 0.2 µm filtered buffer with the lipid/plasmid/detergent solution |
| Note 4: | As an alternative to adding 2X dialysis buffer, the plasmid may be pre-dialyzed against dialysis buffer and the formulation may be diluted to its final volume normal strength dialysis buffer. While this means that there will be a slight difference in the buffer concentration, this does not affect the encapsulation efficiency or resulting particle size. |
| Note 5: | If the volume of the formulation is increased (*i.e*., above 5 mL), add another dialysis change. |
| Note 6: | DEAE-Sepharose columns are often pre-treated by eluting 50 µL of a 10 mg/ml extruded or sonicated 1:1 phosphatidylcholine:cholesterol vesicle formulation (diluted in 2 mL) to block any nonspecific lipid binding to the column. |

To reduce the cationic surface charge of INEX 351 formulations, it may be desirable to reduce the amount of cationic lipid (*i.e.,* DODAC) employed. If the amount of DODAC is changed, the amount of DOPE is changed to maintain the same total amount of lipid. Formulations below 30% DODAC are preferably made in 10 mg total lipid. Dialysis buffer may be changed as in Table 1, below:

**Table 1. Characterization of representative large scale formulations.**

| | Starting | Buffer | Encapsulation | Nicomp particle |
|---|---|---|---|---|
| Conc. | volume | | efficiency | size (nm)^{a} |
| 42.5% | 30 ml | 150 mM NaPO₄, 130 mM NaCl | 49 % | 131 |
| 30% | 12 ml | 150 mM NaPO₄ | 56.8 % | 109 |
| 24% | 30 ml | 130 mM NaPO₄ | 50.7 % | 250 |
| 20% | 15 ml | 105 mM NaPO₄ | 63 % | 178 |

| | | | | |
|---|---|---|---|---|
| ^{a}Nicomp analysis of mean particle size, gaussian dist., volume weighting, before DEAE cleaning and isolation. | | | | |

### Formulation 1.3 (Or, Alternatively, INEX 321)

Lipid-plasmid particles with 10-30% DODAC are also useful in the present invention. These can be formulated, as described above, or as follows.
Lipid stock solutions: Individual stock solutions of each lipid were dissolved in chloroform/methanol (2:1 v/v) to a final concentration of 2 or 20 mg/mL.
OGP solution: 1.0 M OGP solution was prepared in MilliQ grade water.
Citrate buffer: Sodium citrate buffer was used for dialysis to remove detergent from the formulation. The citrate concentrations were varied according to the amount of DODAC. Buffer also contains 150 mM NaCl and 5 mM HEPES at pH 7.4, unless indicated otherwise. In general, a 10X solution was prepared and diluted 1:10 in MilliQ Plus water for dialysis using a graduated cylinder.
Preparation of lipid/DNA/OGP mixture: A typical formulation contained 10 mg of lipid of DODAC/DOPE/PEG-Cer-C8 and 200 µg DNA. Appropriate amounts of stock solutions containing DODAC, DOPE and PEG-Cer-C8 (normally 15 mol% in this study) were mixed in a glass test tube. If the amount of DODAC is changed, the amount of DOPE is changed to maintain a total of 10 mg lipid. The solvent was first removed under a stream of N₂ gas followed by incubation under vacuum for 3-5 h. To the lipid, 0.2 mL of 1 M OGP was added. The suspension was vortexed until the lipid was totally dissolved and the solution became clear. Then a 0.2 mL DNA (1 mg/mL) solution containing 200 µg DNA and 0.6 mL HBS (HEPES buffered saline) or citrate buffer (concentrations designated in Figure 1) were added to a final total volume of 1 mL. If the solution did not become clear, a small amount of OGP (50 µL) was added. The solution was incubated at room temperature for 1 h to allow the components to equilibrate.
Dialysis: Dialysis tubes were soaked in 60% ethanol (or in distilled water if sterilization was not required) for 30 min. The mixture of DNA/lipid/OGP solution was then transferred to the dialysis tube. The sample was dialyzed for 2 days in 2-4 L citrate buffer (concentration as described in Figure 1) with two changes of buffer daily.

After preparation, empty liposomes can be removed by DEAE cleaning and sucrose density centrifugation, as described above. Having been taught the various lipid-plasmid particle formulations suitable for systemic delivery in this example, it would be obvious to one skilled in the art to modify them, for example, for improved plasmid delivery and/or intracellular expression using one or more possible variations. Variations of the following type are suggested: percentage of PEG-lipid; size of PEG; length of hydrophobic (anchor) chain; pH sensitive PEG-lipids; replacement of PEG by ATTA (disclosed in U.S. Patent Application Serial Number 08/996,783, filed December 22, 1997 (bearing TTC Attorney Docket No. 16303-005800 and which is assigned to the assignee of the instant invention); addition of membrane modifying lipids, such as cholesterol or DOPE; use of alternative cationic lipids, such as DMRIE, DOTAP, DOTMA, DODMA, AL-1, *etc.;* use of fusogenic components, such as pH sensitive lipids, peptides (EALA) or polymers (PEAA); use of targeting agents; use of DNA condensing peptides (*i.e*., polylysine or spermine) or polymers (*i.e.,* PEI); use of negatively charged lipids, such as phosphatidylserine; or use of alternative PEG-lipid linkers, such as SPDP or PDPH (disclosed in U.S. Patent Application Serial Number 08/536,584, which is assigned to assignee of the instant invention).

### Formulation 1.4

Formulation 1.4 contains DOPE:DODAC:PEG-Cer-C20 (83:7:10) - mol %. The synthesis protocol is as follows: Aliquot the lipid stock solutions (in ethanol) into an autoclaved, clean, dry round bottom flask. The solution is dried to a lipid film using a rotavap in a 65°C water bath and vacuumed overnight. Add HBS with octylglucopyranoside (OGP) to a final OGP concentration of 200 mM. Swirl the mixture to dissolve the lipid film and, if necessary, heat to 37°C to ensure the lipid is fully dissolved. Plasmid DNA is then added (400 µg /10 mg lipid) to the dissolved lipid films. After incubation at room temperature for 30 min, place the resulting solution in a dialysis bag that has been pre-soaked in filter sterilized distilled H₂O and autoclaved. Dialyze overnight against 20 L of dialysis buffer (5 mM HEPES, 150 mM NaCl, pH 7.4, filter sterilized through a 0.2 micron sterile filter) with two buffer changes.

Nonencapsulated DNA was removed by anion exchange chromatography on a DEAE-Sepharose CL-6B column. Collect the particle suspension as it appears in the eluate, and concentrate using the Amicon difiltration system (YM 30 membrane). Next, empty liposomes were removed using a sucrose density gradient. The gradient was formed by layering 10% sucrose, 5.0% sucrose, and 2.5% sucrose in HBS, pH 7.4. The sample is loaded by floating it on top of the 2.5% sucrose layer and centrifuged at 28,000 rpm for 18 h at 20°C using a Beckman Optima XL-100K ultracentrifuge and an SW-28 rotor. After centrifugation, remove the lower band with a syringe and needle and pool the samples. The sucrose is removed and the sample is concentrated simultaneously using the Amicon system. Filter sterilize the final volume through a 0.2 micron filter. DNA concentration is analyzed by Picogreen assays, lipid concentration by HPLC and particle size by Nicomp analysis.

### Formulation 1.5

This method, set out in PCT patent publication WO 96/40964, is an alternative high-efficiency formulation of the lipid/nucleic acid particle. It is, in essence, a preparation of lipid therapeutic nucleic acid particles in organic solvent. The following stock solutions of lipid are prepared in 100% ethanol: DSPC - 20 mg / mL (20 mol%) = 128.4 µL; Chol - 20 mg / mL (25 mol%) = 113.1 µL; DODAP - 40 mg/mL (45 mol%) = 44.5 µL; PEG-Cer-C20 (or C14) - 50mg/mL (10 mol%) = 67.6 µL.

The lipids are mixed together and the volume is increased to a total volume of 0.400 mL with 100% ethanol. An appropriate volume of 300 mM citrate buffer (pH 3.3) is added to the DNA to a final volume of 600 µL and pH 3.8. Warm the two solutions to 65°C for 2 min. While vortexing the DNA tube, use a Pasteur pipette to add lipid (in ethanol) in a dropwise manner to the DNA solution. The resulting solution will get cloudy and can bubble, but no aggregates should be present. Place the solution in presoaked dialysis tubing (12-14,000 MWCO) and dialyze for 1 h against 300 mM citrate buffer (pH 3.7-4.0). Transfer the dialysis tubing to HBS (pH 7.5) and dialyze for 12 h. Nonencapsulated DNA was removed by anion exchange chromatography using a DEAE-sepharose column equilibrated in HBS. If necessary, the final preparation can be concentrated using the Amicon system (YM 30 membrane). DNA concentration is analyzed by Picogreen assays and the lipid concentration by HPLC.

All of the above lipid-therapeutic nucleic acid formulations have beneficial characteristics that make them suitable for use in the methods of the present invention. Such characteristics include, but are not limited to, the following: First, they are small particles with mean sizes of about 50 about 200 nm and, more preferably, of about 60 to about 130 nm. Most preferably, particles are of a relatively uniform size and have a 12 value of less than 3, more preferably, of less than 1 and, even more preferably, of less than 0.5. Second, they are serum-stable and, thus, are not significantly degraded after exposure to a serum or nuclease assay that would significantly degrade free DNA. Third, they have a nucleic acid to lipid ratio that can be formulated at various levels. For use in the methods of this invention, particles are preferably at least about 3 mg nucleic acid per mmol lipid, more preferably at least about 14 mg per mmol lipid and, most preferably, greater than about 25 mg per mmol. The lipid-nucleic acid particles of the present invention have other advantageous features, such as low nonspecific toxicity, improved biodistribution, therapeutic efficacy and ease of manufacturing.

### Assays for Serum Stability

Lipid/therapeutic nucleic acid particles formulated according to the above noted techniques can be assayed for serum stability by a variety of methods.

For instance, in a typical DNase 1 digestion, 1 µg of DNA encapsulated in the particle of interest is incubated in a total volume of 100 µL of 5 mM HEPES, 150 mM NaCl, 10.0 mM MgC12 pH 7.4. DNase treated samples are treated with either 100 or 10 U of DNase I (Gibco - BRL). 1.0 % Triton X-100 can be added in control experiments to ensure that lipid formulations are not directly inactivating the enzyme. Samples are incubated at 37°C for 30 min after which time the DNA is isolated by addition of 500 µL of DNAZOL followed by 1.0 mL of ethanol. The samples are centrifuged for 30 min at 15,000 rpm in a tabletop microfuge. The supernatant is decanted and the resulting DNA pellet is washed twice with 80% ethanol and dried. This DNA is resuspended in 30 µL of TE buffer. 20 µL of this sample is loaded on a 1.0% agarose gel and subjected to electrophoresis in TAE buffer.

In a typical serum assay, 50 µg of DNA in free, encapsulated, or encapsulated + 0.5% Triton X100 was aliquoted into 1.5 mL Eppendorf tubes. To the tubes were added 45 µl normal murine or human serum, dH₂O (to make final volume 50 µL). The tubes were sealed with parafilm and incubated at 37°C. A sample of the free, encapsulated, or encapsulated + 0.5% Triton X100 not digested by nuclease (standard) was frozen in liquid nitrogen in an Eppendorf tube and stored at -20°C. Aliquots were taken at various time points, added to GDP buffer containing proteinase K (133 µg/mL) and immediately frozen in liquid nitrogen to stop the reaction. Once all of the time points were collected, the samples were incubated at 55°C in a waterbath to activate proteinase K enabling it to denature any remaining exonuclease. Proteinase K digested samples were applied to polyacrylamide gels to assess levels of exonuclease degradation.

Particles disclosed above demonstrate serum stability by showing less than 5% and preferably undetectable amounts of DNA degradation (partial or total) as a result of such treatment, even in the presence of 100 U DNase 1. This compares favorably to free DNA, which is completely degraded, and plasmid/lipid complexes (such as DOTMA or DODAC:DOPE complexes), wherein DNA is substantially (*i.e*., greater than 20%, often 80%) degraded after such treatment.

### EXAMPLE 2

This example illustrates the measurement of the therapeutic effect of lipid formulated ganciclovir on subcutaneous tumors transfected with lipid encapsulated HSV-TK.

| Group | Tumor | Plasmid | Prodrug | Route | Assay | Mice per Group |
|---|---|---|---|---|---|---|
| A | B16 | L018 | PBS | IV | Tumor Volume | 6 C57 |
| B | B16 | L018 | GCV | IV | Tumor Volume | 6 C57 |
| C | B16 | pTK10 | PBS | IV | Tumor Volume | 6 C57 |
| D | B16 | pTK10 | GCV | IV | Tumor Volume | 6 C57 |

The plasmid L018, which is based on pBR322, carries a CMV promoter operably linked to a liciferase gene.

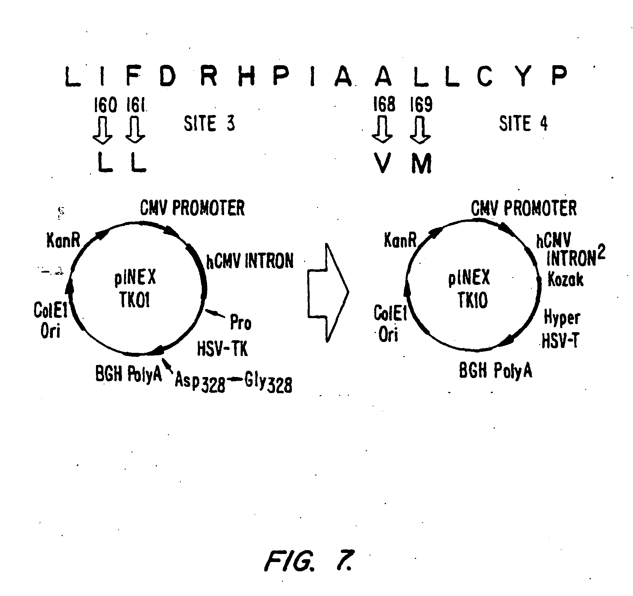

The pINEX-TK10 construct consists of a pBR322 derived plasmid containing a CMV promoter linked to a "hyper" HSV-TK gene, bovine growth hormone polyadenylation sequence and kanamycin resistance gene. "hyper" HSV-TK is a more active form of the HSV-TK gene as disclosed by Black, et al., PNAS (USA), 93:3525-3529 (1996). The plasmid construct employed is set forth in Figure 7.

On day zero, 24 female C57 mice (Harlan Sprague Dawley, Inc., Indianapolis, IN) are seeded sub-cutaneously with 100,000 B16 mice melanoma cells (NCI catalog B16BL-6) in a total volume of 50 µL (groups A, B, C, D). Tumor volume was determined daily by measuring the length, width and height of the tumor with skin calipers as soon as possible and every day thereafter. Groups A to D were treated with 100 µg plasmid of the appropriate lipid-formulated plasmid, formulated according to Example 1, once daily beginning at 9:00 a.m. on day five and on every day following. The plasmid formulation was injected IV in the tail vein in a total volume of 200 µL PBS. Groups B and D were treated with lipid formulated ganciclovir, prepared according to Example 4, once daily beginning at 3:00 p.m. on day five and on every day following. 0.5 mg ganciclovir (~25 mg/kg) were injected IV in the tailvein in a total volume of 200 µL PBS. On day 21, mice were sacrificed. Tumors are collected and weighed.

The results obtained demonstrate that the mice of group D either did not develop tumors, or else developed tumors significantly more slowly than mice of control groups A, B and C.

### EXAMPLE 3

This example illustrates the measurement of therapeutic effect of systemic delivery of treatment with the therapeutic nucleic acid HSV-TK followed by treatment with lipid-formulated ganciclovir on SCID mice having human (SKOV-3) intraperitoneal (IP) tumors.

| Group | Tumor | Plasmid | Prodrug | Route | Assay | Mice per Group |
|---|---|---|---|---|---|---|
| A | SKOV-3 | L018 | PBS | IV | Tumor Volume | 6 C57 |
| B | SKOV-3 | L018 | GCV | IV | Tumor Volume | 6 C57 |
| C | SKOV-3 | pTK010 | PBS | IV | Tumor Volume | 6 C57 |
| D_ | SKOV-3 | pTK010 | GCV | IV | Tumor Volume | 6C57 |

| | | | | | | |
|---|---|---|---|---|---|---|
| * It is noted that the "Route" refers to the delivery of the prodrug. | | | | | | |

On day zero, 24 female C57 mice were seeded intraperitoneally with 5,000,000 SK-OV-3 human ovary adenocarcinoma cells (ATCC HTB-77)) in a total volume of 500 µL (groups A, B, C, D). Groups A to D were treated with 100 µg plasmid of the appropriate lipid-formulated plasmid, formulated according to Example 1, once daily beginning at 9:00 a.m. on day five and on every day following. The plasmid formulation was injected IV in the tail vein in a total volume of 200 µL PBS. Treatment continued for two weeks.

Groups B and D were treated with lipid formulated ganciclovir, prepared according to Example 4, once daily beginning at 3:00 p.m. on day five and on every day following. 0.5 mg ganciclovir (~25 mg/kg) was injected IV in the tailvein in a total volume of 200µL PBS. Mice were monitored for survival. If tumors developed, mice were sacrificed and the tumors collected and weighed. The results obtained demonstrate that the mice of group D either did not develop tumors, or else developed tumors significantly more slowly than mice of control groups A, B and C.

### Example 4

This example illustrates the protocol for the preparation of lipid formulated ganciclovir in a sphingomyelin/cholesterol lipid formulation.

For a 1 mL preparation 100 mg (180 µmole) of lipid was used, of which 55 mole% was sphingomyelin (99 µmoles) and 45 mole% (81 µmoles) was cholesterol (Northern Lipids, Vancouver, BC). Dissolve each lipid in 1 mL of chloroform. Aliquot the required amounts of each lipid into one tube to obtain a 55/45 SM/Chol mixture. Next, add 4500 dpm/µmole of lipid of ¹⁴C-CHE (¹⁴C-cholesteryl hexadecyl ether) and dry the lipid to near dryness under nitrogen. Apply to the lyophilizer overnight and make up a 70/30% solution of HBS/ethanol (HBS is 20 mM Hepes, 145 mM NaCl, pH 7.45). Next, dissolve 100 mg ganciclovir (109 mg ganciclovir-Na, Hoffman LaRoche Ltd.) in 1 mL of 70/30% HBS/ethanol and vortex well. Add 42000 dpm/µmole 3H-GCV (7.5 µL of a 1 Ci/mL stock) and add ganciclovir solution to the lipid film and vortex well. Vortex until the solution appears homogeneous. Freeze-thaw for 5 cycles (liquid nitrogen and 65°C). Warm the cryovial up slightly before putting in the water bath. Next, take 2-10 µL pre-extrusion samples and assay for radioactivity using the dual label program. Take note of the final volume and use this to determine specific activity for both the lipid and GCV. Extrude the sample 2 x through 3 x 100 nm filters at 65°C at 350 psi. At this point, the sample becomes quite viscous. Add 1 mL HBS to the samples and mix by pipetting up and down. Continue extrusion for a total of 10 passes. Take 2-10 µL post-extrusion samples and assay for radioactivity. Hydrate some dialysis tubing (MW cutoff 12,000-14,000) in dH₂0 for 15 min. Next, put the extruded sample in the tubing and dialyze for 1 h against 2 L HBS. Change to fresh buffer and dialyze overnight. Take 2-10 µL samples and assay for radioactivity and determine the percentage loading by comparing the pre-extrusion and post-dialysis ratios of ³H/¹⁴C. For example: ³H/¹⁴C pre-extrusion = 12.0; ³H/¹⁴C post-dialysis = 1.2; 1.2/12.0 x 100% = 10% encapsulation.

### EXAMPLE 5

This example sets forth the protocol for stable transfection of B16 tumor cells with HSV-TK, for use in Examples 6 and 7, as described in SHORT PROTOCOLS IN MOLECULAR BIOLOGY, Third Edition, page 9-13 to 9-15, with the following modifications.

According to the method, the following materials were used. Plasmids: pCMVTKIRESneo is based on plasmid pBR322 and includes a CMV promoter, HSV-TK gene, internal ribosome entry site and neomycin resistance gene. L018 is also based on pBR322, but carrying a CMV promoter and a luciferase gene. First, plate B16 murine melanoma cells in a tissue culture flask (T-75) at 5 x 10⁵ cells/flask in 10 mL MEM media with addition of 10% FBS and Glutamine and grow overnight in CO₂ incubator at 37°C to 70% confluency. Next, aspirate media and feed cells with 3.8 mL fresh media per flask 2 h prior to transfection and prepare plasmid/lipid Lipofectin (GIBCO BRL) aggregate in a polystyrene tube according to manufacturer's instructions. Alternatively, dilute plasmid to 20 µg/mL in sterile distilled water. Use Luciferase L018 plasmid as a control for negative selection in Geneticin (G418), use Thymidine Kinase (neomycin) 20A for TKneo stable cells; dilute lipid to 1 mM in sterile distilled water; dilute lipid to charge ratio 1 in sterile distilled water (1.2 mL lipid/8 mL water); add volume of DNA (20 mg/mL) to equivalent volume of lipid (CR1) dropwise while vortexing; and incubate DNA/lipid complex for 30 min at room temperature. Next, slowly add 1.2 mL DNA/lipid complex/T75 flask, mix gently and incubate 24 h in CO₂ incubator at 37°C (complex is diluted 1:4 in media). Aspirate media, wash with PBS buffer and split each T75 flask into 2-100 x 20 mm tissue culture dishes. Next, 24 h after plating into dishes, add the selective agent, Geneticin(G418), at the appropriate concentration to kill nontransfected cells, yet allow cells with transfected TKneo to stay alive. The Luciferase control cells should die. Every 2-3 days change the media to remove dead cell debris. Within 10 days clones are visible on bottom of 100 mm dish which are neomycin resistant and TK positive. Scrape clones into 1 mL media in 24-well plate and expand up into T-75 flask. Cells that stably express TK can then be used for local, regional or systemic injection into mice. To screen identified clones for TK expression, plate 2000 cells/well in 96 well plate in 150 µL volume and grow 48 h in CO₂ incubator at 37°C; add the specific prodrug for TK, ganciclovir, in a dilution series across the plate made up at 2.5 x concentrated and add 100 µL/well (add to 150 µL volume); incubate 3 days in CO₂ incubator at 37°C; aspirate media from wells and add Alamar Blue as per manufacturers instructions (Biosource International) (1:10 dilution in media); and 100 µL/well and incubate for 1, 2, 4 h and read plate at time intervals on fluorescent plate reader (550, 595 nm; 750V; 70 offset; 100 ms integration time).

### EXAMPLE 6

This example illustrates the effects of systemically delivered lipid-formulated ganciclovir on tumor growth in mice having B16 intradermal tumors stably transfected with HSV-TK.

| Group | Tumor | Prodrug | Route | Assay | Timepoint | Mice per Group |
|---|---|---|---|---|---|---|
| | | | | | (assay) | |
| A | B16 | PBS | IV | Tumor Volume | DAILY | 8 C57 |
| B | B16 TK | PBS | IV | Tumor Volume | DAILY | 8 C57 |
| C | B16 | LIPO-GCV | IV | Tumor Volume | DAILY | 8 C57 |
| D | B16 TK | LIPO-GCV | IV | Tumor Volume | DAILY | 8 C57 |

| | | | | | | |
|---|---|---|---|---|---|---|
| * It is noted that the "Route" refers to the delivery of the prodrug, *i.e.,* gangciclovir (GCV). | | | | | | |

32 female C57 mice (Harlan Sprague Dawley, Inc., Indianapolis) were seeded intradermally in groups A and C with B16 parental control cells (B16), and in groups B and D with B 16 tumor cells stably transfected and expressing HSV-TK (B16 TK) (prepared as previously described) at a dose of 150,000 cells in a total volume of 50 µL phosphate buffered saline on day zero. Intradermal tumor volume was determined daily by measuring the length, width and height of the tumor with skin calipers as soon as possible and every day thereafter.

The mice were treated with the ganciclovir prodrug, lipid formulated as in Example 4, once every two days beginning on day four and on every second day following. The ganciclovir dosage of 0.5 mg (~25 mg/kg) was injected IV in a total volume of 200 µL PBS (phosphate buffered saline). Mice received a total of nine treatments. On day 21, mice were sacrificed. Tumors were collected and weighed prior to fixation for sectioning.

Intradermal tumors stably transfected with HSV-TK showed no measurable growth when treated systemically with lipid formulated ganciclovir. Untreated B16 tumors, and treated B 16 tumors without TK, were not affected by the drug.

### EXAMPLE 7

This example was carried out to determine the effect of lipid formulated ganciclovir on TK gene expression in B16 tumor cells stably transfected with HSV-TK and implanted intravenously.

| Group | Tumor | Prodrug | Route | Assay | Timepoint | Mice per Group |
|---|---|---|---|---|---|---|
| | | | | | (assay) | |
| A | B16 TK | PBS | IV | Tumor Volume | DAILY | 8 C57 |
| B | B16 TK | LIPO-GCV | IV | Tumor Volume | DAILY | 8 C57 |

| | | | | | | |
|---|---|---|---|---|---|---|
| * It is noted that the "Route" refers to the delivery of the prodrug, *i.e*., gangciclovir (GCV). | | | | | | |

16 female C57 mice were seeded with B16 tumor cells stably expressing HSV-TK by tail vein injection at a dose of 150,000 cells in a total volume of 200 µL phosphate buffered saline on day zero. The mice were treated with the ganciclovir prodrug, lipid formulated as in Example 4, once every day beginning on day two and on the two days following. The ganciclovir dosage of 0.5 mg (~25 mg/kg) was injected IV in a total volume of 200 µL PBS (phosphate buffered saline). Mice received a total of three treatments. On day 21, mice were sacrificed and tumors were scored. Livers, lungs, spleen and pancreas were photographed. There was a significant reduction in both size and number of metastatic tumor nodules.

### EXAMPLE 8

This example illustrates gene expression in distal metastatic tumors using Formulation 1.1 lipid plasmid particles.

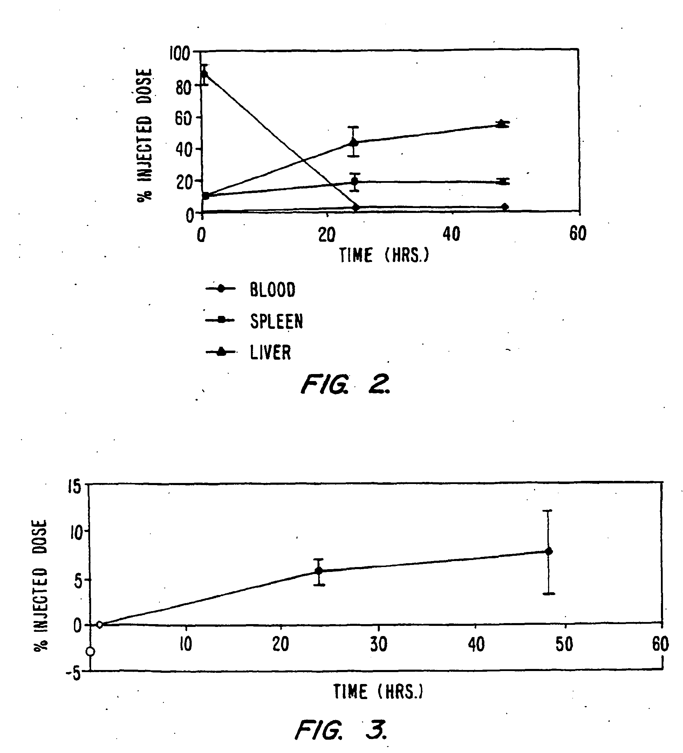

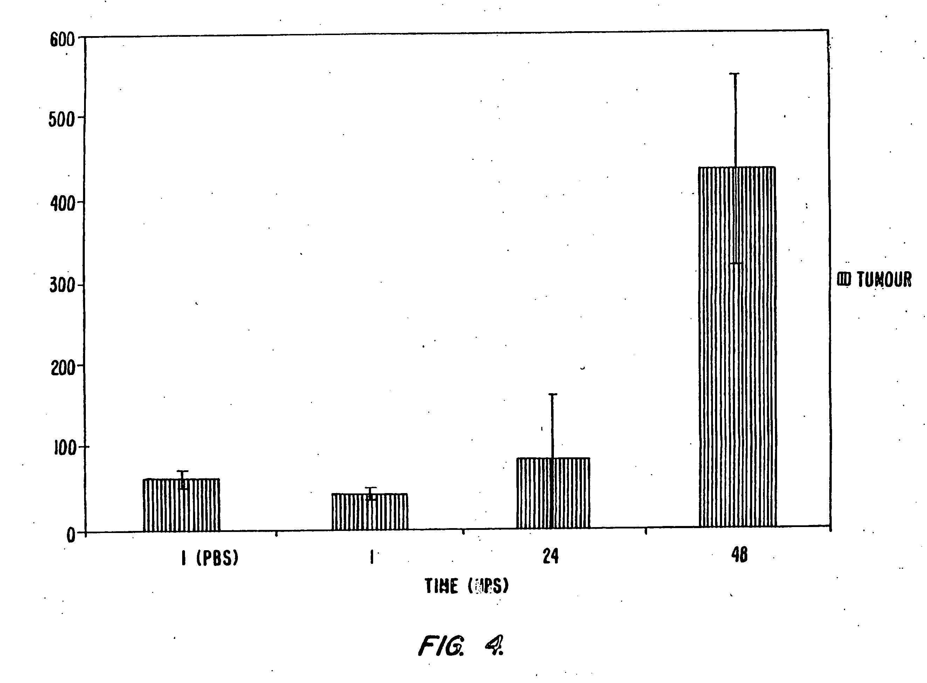

On day zero, C57BL/6 mice (Harlan Sprague Dawley, Inc., Indianapolis, IN) are seeded with 300,000 LL/2 (Mouse Lewis Lung Carcinoma) cells (ATCC CRL-1642) by intravenous/tail vein injection with total volume 200 µL. On day 10, the mouse is intravenously injected with formulation 1.1 plasmid-lipid particles. The particles carry plasmid L018, which is a standard construct containing the luciferase gene linked to the CMV promoter. At various time points after plasmid injection, mice are sacrificed, and organs and tumors are quickly frozen in liquid nitrogen, then stored at -70°C. Organs/tumors are assayed for the luciferase gene to demonstrate delivery to the organ/tumor site. Biodistribution results for organs are shown in Figure 2. Accumulation at the tumor site is illustrated in Figure 3. Southern blot data shows presence of intact plasmid at the tumor site increasing to at least 96 h. Cell protein from organs/tumors is also prepared and assayed for luciferase according to standard techniques. A time course of gene product activity at distal (metastatic) tumor sites is demonstrated in Figure 4.

### EXAMPLE 9

This example illustrates the systemic vector delivery and gene expression in an *in vivo* human tumor.

SCID mice (Harlan Sprague Dawley, Inc., Indianapolis, IN) were seeded with 1 x 10⁶ LS 180 human colon adenocarcinoma cells (ATCC CL-187) by subcutaneous injection on day zero. On day 11, mice in groups A, B and C were injected intravenously with indicated doses of L018 plasmid in lipid formulation 1.1, in 200 µL total volume. On day 17, mice in group D and E were injected intravenously with L018 plasmid in lipid Formulation INEX 320, using C8 or C20 PEG-Cer) according to Example 1, in 200 µL total volume. At the times indicated after plasmid injection, mice were sacrificed and organs (liver, spleen and lungs) and tumors were harvested. Expression of the enzyme Luciferase was assayed according to standard techniques on all samples.

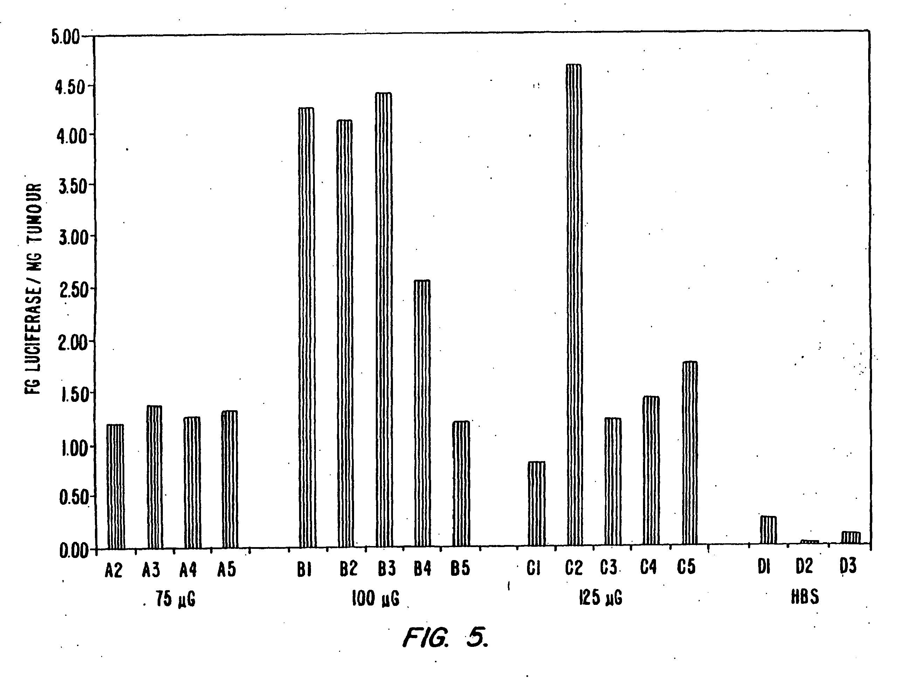

The data obtained demonstrates the excellent transfection and expression of the reporter gene Luciferase achieved in an *in vivo* human tumor using the lipid-nucleic acid particles of the present invention *(see,* Figure 5).

| Group | Formulation | Assay | Time Point | Mice per group |
|---|---|---|---|---|
| A | 1.1 (75 µg) | Luciferase | 48 h | 5 |
| B | 1.1(100 µg) | Luciferase | 48 h | 5 |
| C | 1.1 (125 µg) | Luciferase | 48 h | 5 |
| D | 320 (100 µg) | Luciferase | 24 h | 4 |
| E | 320 (100 µg) | Luciferase | 48 h | 4 |
| F | PBS | Luciferase | 48 h | 1 |

### EXAMPLE 10

This example demonstrates systemic delivery and expression at an *in vivo* tumor site of a vector containing the HSV-TK gene, using a lipid-nucleic acid particle prepared according to Example 1.

C57 mice are intraperitoneally seeded with 100,000 B16 tumor cells in a total volume of 200 µL PBS on day zero. On day 14, test mice are injected with Formulation 1.2 (100 µg DNA in 500 µL PBS) prepared according to Example 1. The plasmid vector used is pINEX-TK10 (Figure 7) as described earlier. 24 h later, mice are sacrificed, and tumors are isolated, fixed within 5 min, and prepared in paraffin sections using standard techniques. The expression of the HSV-TK gene at the distal tumor site is assayed by in situ RNA/RNA hybridization using techniques standard in the art. One such technique is summarized below.

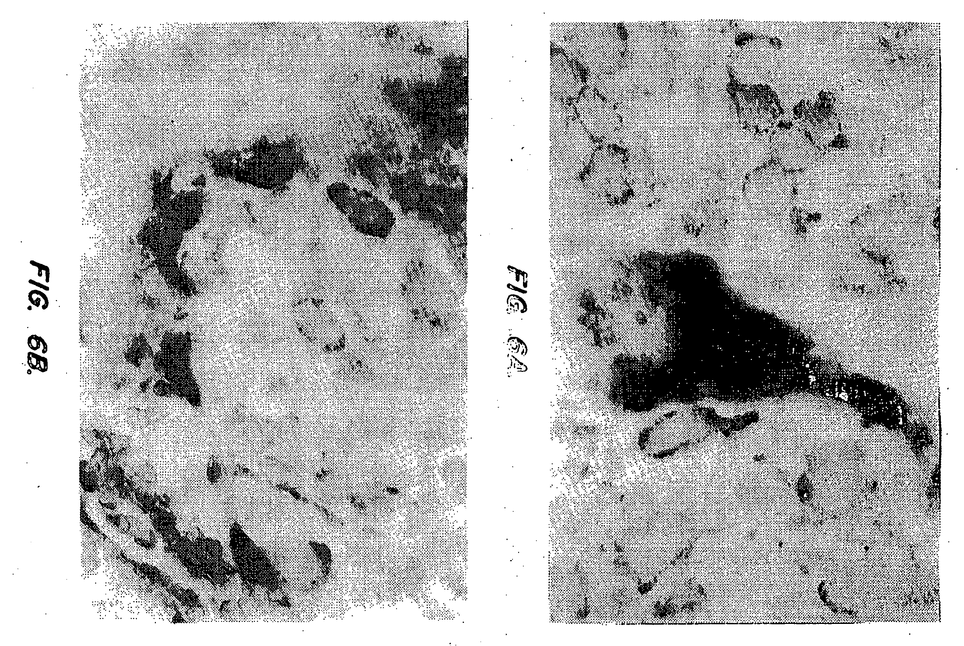

The pattern of HSV-TK gene expression within peritoneal tumors is demonstrated in Figures 6(A) and (B). In all cases of gene expression, positive signal is observed as a cellular content of B16 cells or endothelial cells. Positive stained cells are localized in proliferative zone associated with blood vessels or peripheral area.

### EXAMPLE 11

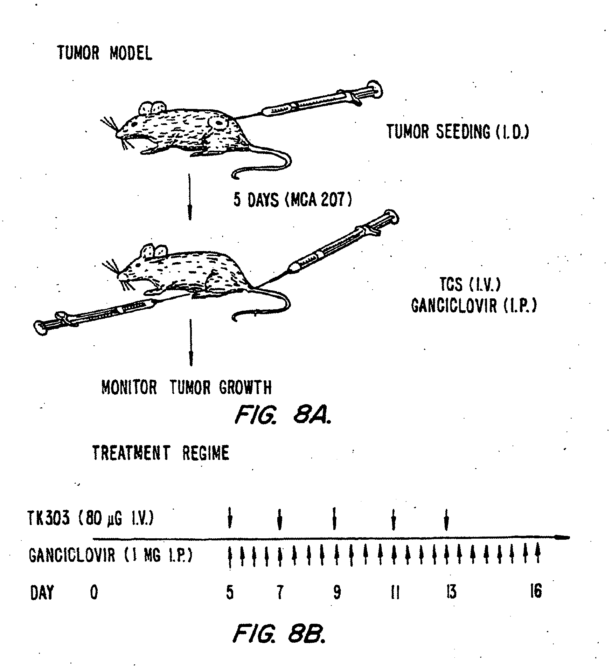

This example describes the treatment of tumors using the method of the invention. In particular, this example demonstrates the effect of pINEX-TK10 in Formulation 1.1, in inhibiting the growth of MCA-207 tumors following treatment with ganciclovir. The general method is set out in Figure 8.

| Group | Formulation | GCV | Route* | Assay | Timepoint | # of Mice |
|---|---|---|---|---|---|---|
| A | PBS | PBS | IP | Volume/CTL | --- | 6 C57 |
| B | Empty 1.1 | PBS | IP | Volume/CTL | --- | 6 C57 |
| C | 1.1 TK | PBS | IP | Volume/CTL | --- | 6 C57 |
| D | 1.1 TK | GCV | IP | Volume/CTL | --- | 6 C57 |

| | | | | | | |
|---|---|---|---|---|---|---|
| *It is noted that the "Route" refers to the delivery of the prodrug, *i.e*., gangciclovir. | | | | | | |

24 female C57 mice were seeded with 100,000 MCA-207 fibrosarcoma tumor cells (provided by S. Rosenberg, National Cancer Institute, Frederick/Bethesda, MD) by intra-dermal injection on day zero. The tumor cells had been cultivated and prepared according to standard techniques using RPMI media with 10% Fetal Bovine Serum *(see* for example Current Protocols in Molecular Biology). Beginning on day 5, all animals were treated with the lipid/therapeutic nucleic acid formulation listed in the chart, *supra.* The formulation was delivered intravenously through the tail vein. 80 µg of pINEX-TK10 DNA were injected in a total volume of 200 µL. Treatments were administered on days 5, 7, 9, 11 and 13.

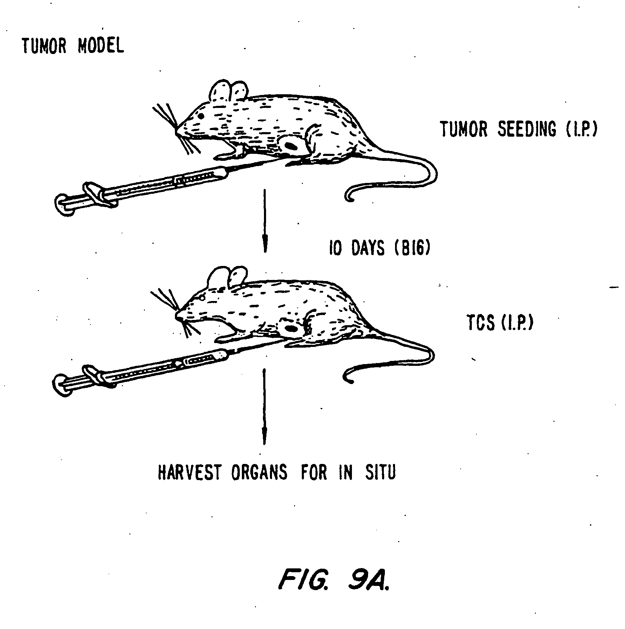

Beginning on day 5, all animals were treated with ganciclovir twice daily. 1 mg (-50 mg/kg) were injected intra-peritoneally in a total volume of 200 µL PBS. Treatments continued twice daily for 12 days *(see,* Figure 9(A)). Mice were monitored for tumor growth.

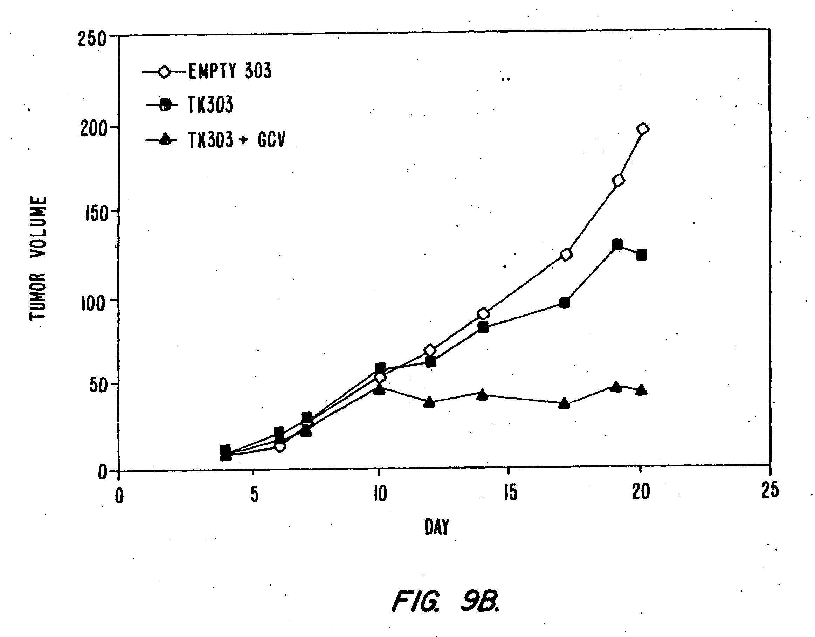

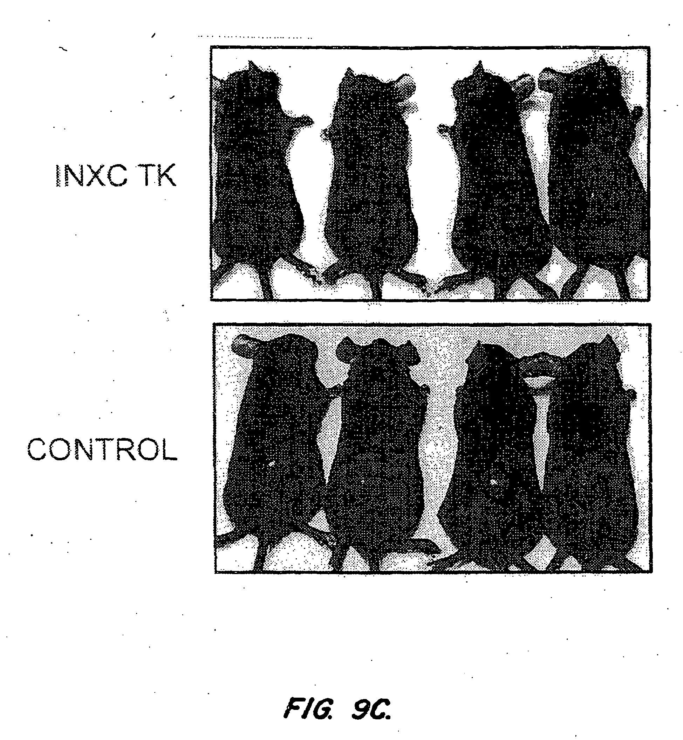

Figure 9(B) sets out in more quantitative terms the effect of the treatments. Mice treated with HSV-TK in formulation 1.1 have greatly reduced tumors compared to control treated mice. Not shown is data of control mice which demonstrates that treatment with empty liposomes and ganciclovir has no effect on tumor reduction. Figure 9(C) demonstrates the effect of the treatment on test mice in comparison with control mice at day 16 after tumor inoculation.

### EXAMPLE 12

This example illustrates the protocol for RNA/RNA *in situ* hybridization of *in vivo* tumors transfected by systemically delivered plasmid.

Tumors were prepared for *in situ* investigation by paraffin embedding and staining. Specifically, peritoneal tumors are collected into 4% paraformaldehyde/PBS fixative (Sigma Chemical Co.) and fixed overnight at 4°C. Serial 5-µm sections are prepared after the samples have been dehydrated in graded ethanol solutions, cleared in chloroform and embedded in paraffin wax (Paraplast Plus, Fisher).

When ready to be used, prepared samples were treated with two changes of xylene for 10 min, each followed by rehydration in graded ethanol solutions to 50% ethanol. Samples are prehybridized by standard rinsing, incubation with 0.1 M triethanolamine (TEA) buffer, pH 8.0, containing 0.25% (v/v) acetic anhydride, followed by treatment at 56°C for at least 60 min in hybridization buffer containing: 40% deionized formamide, 10% dextran sulfate, 1 x Denhardt's solution (0.02% Ficoll, 0.02% polyvinylpyrrolidone, 10 mg/mL RNase-free bovine serum albumin), 4 x SSC, 10 mM DTT, 1 mg/mL yeast t-RNA, and 1 mg/mL denatured and sheared salmon sperm DNA.

Labelling of RNA probe by *in vitro* transcription of DNA was as follows. The fragment of 599 bp (532 - 1131) from HSV-TK (pTK10) was cloned into KpnI and BamHI sites of the vector pGEM-7Zf(+) (pTK11). The plasmid is cloned by standard techniques and prepared using Qiagene 500 (Qiagen, Inc.). For the anti-sense probe, this plasmid is linearized by cutting it with KpnI at the original 5' end of the cDNA HSV-TK and purified. The same logic is used for sense (control) probe (*i.e.,* cut at the side of the 3-end of insert by BssHII or BamH or SacI). The plasmid is purified by ethanol precipitation. The following are then mixed in a 1.5 mL sterile RNase free microcentrifuge tube on ice: 4 µL (4 µg) purified, linearized plasmid DNA, 5 µL of 10 x concentrated DIG RNA Labeling Mix (supplied by manufacturer), 10 µL 5 x concentrated Transcription Buffer (400 mM Tris-HCl (pH 8.0, 20°C), 60 mM MgCl2, 100 mM Dithiothreitol (DTT), 20 mM spermidine], 2 µL RNasin, 3 µL RNA polymerase (SP6 for antisense or T7 for sense), and sterile, redistilled water to make a total reaction volume of 50 µL.

The components are mixed and centrifuged briefly, and then incubated for 2 h at 37°C (for T7 RNA polymerase) or at 40°C (for SP6 polymerase). After incubation, add 3 µL DNase I, RNase free and 1 µL RNasin to the tube and incubate for 15 min at 37°C. Then add 2.5 µL 0.5 M EDTA (pH 8.0) to the tube to stop the polymerization reaction.

The labeled RNA transcript is precipitated by performing the following steps. Add to the reaction tube 6.25 µL 4 M LiCl and 180 µL prechilled (-20°C) 100% ethanol incubate overnight at -70°C. Centrifuged the tube (at 13,000 x g) for 15 min at 4°C. Discard the supernatant. Wash the pellet with 50 µL ice-cold 70% (v/v) ethanol. Centrifuge the tube (at 13,000 x g) for 5 min at 4°C. Discard the supernatant and dry the pellet at room temperature. Dissolve the RNA pellet for 30 min at 37°C or (R.T.) in 20 µL DEPC (diethylpyro-carbonate)-treated, sterile, redistilled water, added 20 µL deionized formamide and 1 µL RNasin. Keep transcript at -20°C or -70°C.

An accurate quantification of DIG-labeled RNA obtained in the labeling reaction is most important for optimal and reproducible results in various membrane or in situ hybridization techniques. Too high of a probe concentration in the hybridization mix usually causes background, while too low of a concentration leads to weak signals. The estimation of yield can be performed in a side by side comparison of the DIG-labeled sample nucleic acid with a DIG-labeled control, that is provided in the labeling kits. Dilution series of both are prepared and spotted on a piece of membrane. Subsequently, the membrane is colorimetrically detected. Direct comparison of the intensities of sample and control allows the estimation of labeling yield.

The hybridization reaction is then performed. Drain pre-hybridization buffer from the pre-hybridized slides and overlay each section with 200 µL of hybridization buffer containing 0.2-1 ng of digoxigenin-labeled RNA probe (0.2 ng/µL). Cover samples with a 24 x 30 mm hydrophobic plastic coverslip. Incubate sections at 56°C overnight in a humid chamber. Washes can include an RNAse step which reduces the background, but decreases the signal as well. It is important to keep the tissue sections moist at all times during washing. Wash the slides in 2xSSC at 55°C for 30 min; wash in 50% formamide, 2xSSC at 65°C for 30 min; wash in 2xSSC 3 times at 37°C for 5 min each; wash in RNase 10 µg/mL, 1xwashing solution at 37°C for 30 min; wash in 50% formamide, 2xSSC at 65°C for 30 min; wash in 2xSSC at 37°C for 15 min; wash in 02xSSC 5 times at 37°C for 5 min each.

After hybridization, cells are incubated DIG-specific antibody. Wash the slides in TBS at RT for 30 min. Incubate sections with blocking solution (TBS and 2% goat serum) at RT for 1 h. Decant blocking solution and incubate sections with goat anti-DIG-alkaline phosphatase (Fab fragment) dilution 1:500 at RT for 1 h. Wash the slides in TBS at RT for 30 min. Wash the slides in substrate buffer (100 mM Tris-HCl (pH 9.5), 100 mM NaCl, 50 mM MgCl2) at RT for 30 min. Prepare a color solution containing: 10 mL substrate buffer, 50 µL NBT (nitroblue tetrazolium) and 37 µL BCIP. Slides are immersed in color solution at room temperature for 1-2 h or at 4°C for 2-3 days. Slides are washed with water and observed by light microscopy. Results are shown in Figures 6(A) and (B).

### EXAMPLE 13

This example illustrates the treatment of tumors using the methods and compositions of the invention. In particular, this example demonstrates the long-term survival of mice bearing MCA-207 tumors that were treated with a course of intravenous pINEX-TK10 in Formulation 1.1 combined with intra-peritoneal injections of ganciclovir (GCV).

The pINEX-TK10 construct, as described above, was formulated in Formulation 1.1, as described above. C57 mice were seeded with 100,000 MCA-207 fibrosarcoma tumor cells (provided by S. Rosenberg, National Cancer Institute, Frederick/Bethesda, MD) by intra-dermal injection on day zero. The tumor cells had been cultivated and prepared according to standard techniques using RPMI media with 10% Fetal Bovine Serum *(see* for example Current Protocols in Molecular Biology).

There were a total of 20 mice in each of the treatment groups and the groups were treated as follows: Group I, ganciclovir alone (GCV); Group II, empty SPLPs and ganciclovir (Empty SPLP + GCV); Group III, formulated plasmid alone (INXC-gTK); and Group IV, formulated plasmid together with ganciclovir (INXC-gTK + GCV). The lipid/therapeutic nucleic acid formulation was delivered intravenously through the tail vein. 80 µg of pINEX-TK10 DNA were injected in a total volume of 200 µL PBS. Treatments with the lipid/therapeutic nucleic acid formulation were administered on days 5, 7, 9, 11 and 13. Animals were treated with ganciclovir twice daily. 1 mg (~50 mg/kg) were injected intra-peritoneally in a total volume of 200 µL PBS. Treatments continued twice daily for 12 days. Mice were monitored for long-term survival.

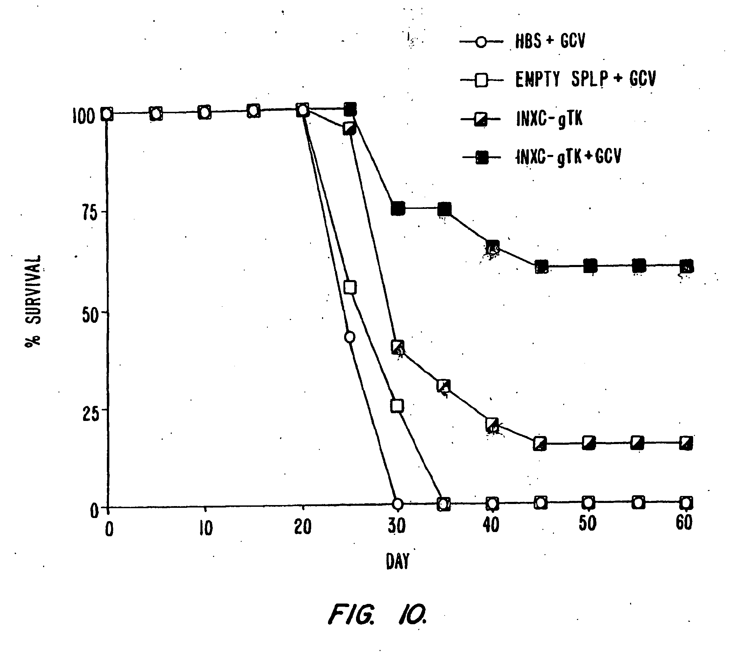

Figure 10 sets out in quantitative terms the effect of the treatments. Mice treated with HSV-TK in Formulation 1.1 together with ganciclovir have greatly increased long-term survival. Only modest effects were observed following treatment with HSV-TK in formulation 1.1 alone compared to untreated controls. Such results demonstrate the successful use of a nonviral gene transfer system that can produce regression in distal tumors following intravenous delivery.

### EXAMPLE 14

This example illustrates the efficacy of systemic delivery of TK303 in the BALB/c CT26 tumor model, *i.e.,* a colorectal tumor model.

| Group | Formulations | GCV | Route | Assay | # of Mice |
|---|---|---|---|---|---|
| A | HBS | PBS | IP | Volume / CTL | 8 BALB/c |
| B | Empty 303 | PBS | IP | Volume / CTL | 8 BALB/c |
| C | Empty 303 | GCV | IP | Volume / CTL | 8 BALB/c |
| D | 303 TK | PBS | IP | Volume / CTL | 8 BALB/c |
| E | 303 TK | GCV | IP | Volume / CTL | 8 BALB/c |

| | | | | | |
|---|---|---|---|---|---|
| Empty 303 is Formulation INEX 303 (Example 1) with no DNA; 303 TK is Formulation INEX 303 containing pTK10 (Figure 7). | | | | | |

On day zero, 48 female BALB/c mice were seeded with 100,000 CT26 tumor cells (ID) using the protocol described in the above examples. Beginning on day 5, all animals were treated with formulation delivered IV through the tail vein. 50 µg of DNA was injected IV in a total volume of 200 µL PBS. Treatment was continued every other day for the next eight days.

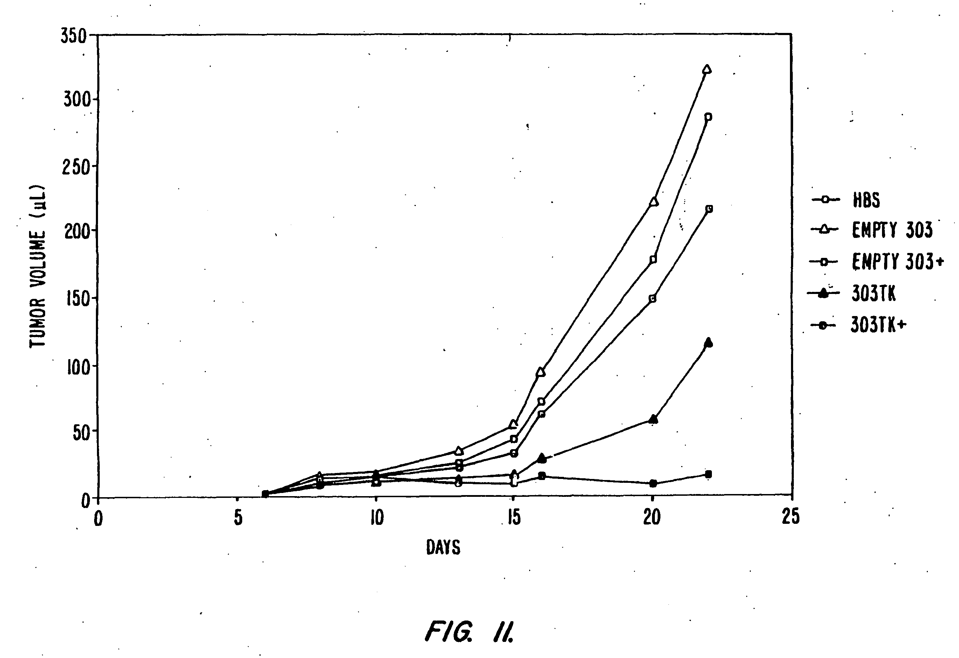

Groups C and E were treated with lipid formulated ganciclovir once daily on day five and on every day following. 1.5 mg (~75 mg/kg) was injected IV in the tail vein in a total volume of 300 µL PBS. Mice were monitored for survival. If tumors developed, mice were sacrificed and the tumors collected and weighed. The results obtained demonstrate that the mice of Group E either did not develop tumors or else developed tumors significantly more slowly than the other mice *(see,* **Figure 11**).

### EXAMPLE 15

This example illustrates the efficacy of systemic delivery of TK303 in the BALB/c CT26 tumor model, *i.e.,* a colorectal tumor model.

| Group | Formulation | GCV | Route | Assay | #of Mice |
|---|---|---|---|---|---|
| A | HBS | PBS | IP | Volume | 8 BALB/c |
| B | Empty 303 | PBS | IP | Volume | 8 BALB/c |
| C | Empty 303 | GCV | IP | Volume | 8 BALB/c |
| D | 303 TK FS* | PBS | IP | Volume | 8 BALB/c |
| E | 303 TK FS* | GCV | IP | Volume | 8 BALB/c |
| F | 303 TK | PBS | IP | Volume | 8 BALB/c |
| G | 303 TK | GCV | IP | Volume | 8 BALB/c |

| | | | | | |
|---|---|---|---|---|---|
| * The vectors used in this experiment were as follows: "TK" refers to pTK10, which has the thymidine kinase coding sequence in its proper reading frame, such that thymidine kinase protein is expressed. "TK FS" has the thymidine kinase coding sequence out of frame relative to the translation initiation codon so that no thymidine kinase protein is produced. | | | | | |

On day zero, 65 female BALB/c mice were seeded with 100,000 CT26 tumor cells (ID) using the protocol described in the above examples. Beginning on day 5, all animals were treated with formulation delivered IV through the tail vein. 50 µg of DNA was injected IV in a total volume of 200 µL PBS. Treatment was continued every other day for the next eight days.

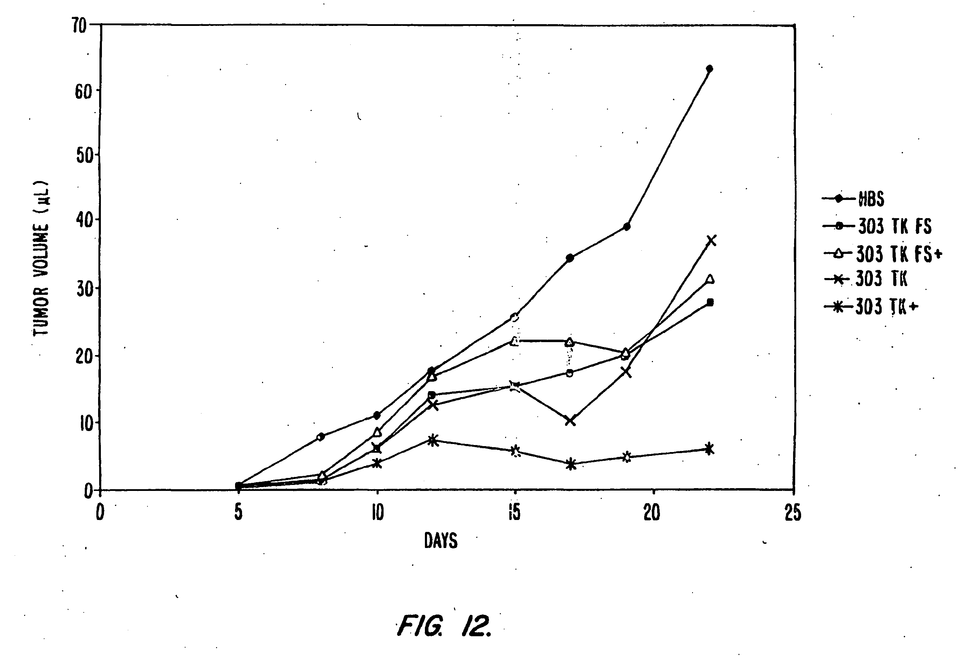

Groups C, E and G were treated with lipid formulated ganciclovir once daily on day five and on every day following. 1.0 mg (~50 mg/kg) was injected IV in the tail vein in a total volume of 200 µL PBS. Mice were monitored for survival. If tumors developed, mice were sacrificed and the tumors collected and weighted. Mice in Group G, which received GCV and the lipid-formulaied TK construct, and thus expressed the TK protein, exhibited a marked reduction in tumor growth rate *(see,* **Figure 12****).**

It is to be understood that the above description is intended to be illustrative and not restrictive. Many embodiments will be apparent to those of skill in the art upon reading the above description. The scope of the invention should, therefore, be determined not with reference to the above description, but should instead be determined with reference to the appended claims, along with the full scope of equivalents to which such claims are entitled.

## Claims

1. Use of a serum-stable nucleic acid-lipid particle comprising a nucleic acid portion that is fully encapsulated within the lipid portion, in the manufacture of a medicament for systemic treatment of a metastatic neoplasia in a mammal by intravenous injection at an injection site that is physically separated from said neoplasia in said mammal, wherein said nucleic acid is a therapeutic nucleic acid.

2. Use in accordance with claim 1, wherein said nucleic acid comprises an expressible gene.

3. Use in accordance with claim 2, wherein said expressible gene encodes a member selected from the group consisting of therapeutic polypeptides and therapeutic polynucleotides.

4. Use in accordance with claim 3, wherein said gene is a member selected from the group consisting of genes encoding suicide enzymes, toxins and ribozymes.

5. Use in accordance with claim 2, wherein said gene encodes a member selected from the group consisting of herpes simplex virus thymidine kinase (HSV-TK), cytosine deaminase, xanthine-guaninephosphoribosyl transferase, purine nucleoside phosphorylase, cytochrome P450 2B1 and analogs thereof.

6. Use in accordance with claim 2, wherein said gene is exogenous to the mammal.

7. Use in accordance with claim 2, wherein said gene is endogenous to the mammal.

8. Use in accordance with claims 2, wherein said gene encodes a member selected from the group consisting of proto-oncogenes, cytokines, immune stimulatory proteins and anti-angiogenic proteins.

9. Use in accordance with claim 2, wherein said gene is a member selected from the group consisting ofIL-2. TL-12, IL-15 and GM-CSF.

10. Use in accordance with claim 2, wherein a therapeutically effective amount of said gene is accumulated at said neoplasia.

11. Use in accordance with claim 1, wherein said nucleic acid-lipid particle comprises a protonatable lipid having a pKa in the range of about 4 to about 11.

12. Use in accordance with claim 11, wherein said protonatable lipid is a member selected from the group consisting of DODAC, DODAP, DODMA, DOTAP, DOTMA, DC-Chol, DMRIE, DSDAC and mixtures thereof.

13. Use in accordance with claim 1, wherein said nucleic acid-lipid particle comprises a lipid conjugate that prevents aggregation during formulation.

14. Use in accordance with claim 13, wherein said lipid conjugate is a member selected from the group consisting of PEG-lipids and PAO-lipids.

15. Use in accordance with claim 13 wherein said lipid conjugate is reversibly associated with an outer lipid monolayer, and wherein said lipid conjugate exchanges out of said outer lipid monolayer at a rate faster than PEG-CerC20.

16. Use in accordance with claim 1, wherein said nucleic acid-lipid particle is substantially devoid of detergents and organic solvents.

17. Use in accordance with claim 1, wherein a therapeutically effective amount of said nucleic acid-lipid particle accumulates at said neoplasia.

18. Use in accordance with claim 1, wherein a therapeutic effect is detected at the site of said neoplasia.

19. Use in accordance with claim 17, wherein said therapeutically effective amount comprises greater than about 0.5% of an administered dose.

20. Use in accordance with claim 1, wherein said nucleic acid-lipid particle has a diameter of about 50 nm to about 200 nm.

21. Use in accordance with claim 20, wherein said nucleic acid-lipid particle has a diameter of about 60 nm to about 130 nm.

22. Use in accordance with claim 20, wherein said nucleic acid-lipid particles are of a uniform size.

23. Use in accordance with claim 1, wherein said nucleic acid-lipid particle has a nucleic acid to lipid ratio of greater than about 3 mg nucleic acid to mmole of lipid.

24. Use in accordance with claim 23, wherein said particle has a nucleic acid to lipid ratio of greater than about 14 mg nucleic acid to mmole of lipid.

25. Use in accordance with claim 23, wherein said particle has a nucleic acid to lipid ratio of greater than about 25 mg nucleic acid to mmole of lipid.

26. Use in accordance with claim 1, wherein said nucleic acid remains at least 90% intact when said particle containing about 1 µg DNA is treated with about 100 U DNAse 1 in digestion buffer at 37°C for 30 min.

27. Use in accordance with claim 1, wherein said medicament is for use with a chemotherapeutic agent.

28. Use in accordance with claim 1, wherein said injection is performed at least once per eight weeks.

29. Use of
a) a serum-stable nucleic acid-lipid particle encoding a gene-product comprising a nucleic acid that is fully encapsulated within the lipid and
b) a first compound which is processed by said gene-product into a second compound, wherein a neoplastic cell is more sensitive to said second compound than said first compound
in the manufacture of medicaments for simultaneous or sequential use in treating a metastatic neoplasia by sensitizing said neoplastic cell to said first compound, wherein said medicament comprising a nucleic acid-lipid particle is administered systemically by intravenous injection at an injection site that is physically separated from said neoplastic cell, and wherein said particle transfects said neoplastic cell.

30. Use in accordance with claim 29 wherein said first compound is formulated in a lipid.

31. Use in accordance with claim 29 wherein said gene product is a member selected from the group consisting of therapeutic polypeptides and therapeutic polynucleotides.

32. Use in accordance with claim 29 wherein said gene product is a member selected from the group consisting of suicide enzymes, toxins and ribozymes.

33. Use in accordance with claim 29 wherein said gene product is a member selected from the group consisting of herpes simplex virus thymidine kinase (HSV-TK), cytosine deaminase, xanthine-guaninephosphoribosyl tranferase, purine nucleoside phosphorylase, cytochrome P450 2B1 and analogs thereof.

34. Use in accordance with claim 29 wherein a therapeutic effect is detected at the site of said neoplastic cell.

## Patentansprüche

1. Verwendung eines Serum-stabilen Nukleinsäure-Lipidpartikels, umfassend einen Nukleinsäure-Anteil, der vollständig eingekapselt ist innerhalb des Lipidanteils, in der Herstellung eines Medikaments für die systemische Behandlung einer metastatischen Neoplasie in einem Säuger durch intravenöse Injektion an einer Injektionstelle, die physisch getrennt ist von der Neoplasie in dem Säuger, wobei die Nukleinsäure eine therapeutische Nukleinsäure ist.

2. Verwendung nach Anspruch 1, wobei die Nukleinsäure ein exprimierbares Gen umfasst.

3. Verwendung nach Anspruch 2, wobei das exprimierbare Gen für ein Mitglied codiert, ausgewählt aus der Gruppe, bestehend aus therapeutischen Polypeptiden und therapeutischen Polynukleotiden.

4. Verwendung nach Anspruch 3, wobei das Gen ein Mitglied ist, ausgewählt aus der Gruppe, bestehend aus Genen, die für Suizidenzyme, Toxine und Ribozyme codieren.

5. Verwendung nach Anspruch 2, wobei das Gen für ein Mitglied codiert, ausgewählt aus der Gruppe, bestehend aus Herpes simplex-Virus-Thymidinkinase (HSV-TK), Cytosindeaminase, Xanthin-Guanin-Phosphoribosyltransferase, Purinnukleosidphosphorylase, Cytochrom P450 2B1 und Analoga davon.

6. Verwendung nach Anspruch 2, wobei das Gen exogen für den Säuger ist.

7. Verwendung nach Anspruch 2, wobei das Gen endogen für den Säuger ist.

8. Verwendung nach Anspruch 2, wobei das Gen für ein Mitglied codiert, ausgewählt aus der Gruppe, bestehend aus Proto-Onkogenen, Zytokinen, immunstimulatorischen Proteinen und anti-angiogenen Proteinen.

9. Verwendung nach Anspruch 2, wobei das Gen ein Mitglied ist, ausgewählt aus der Gruppe, bestehend aus IL-2, IL-12, IL-15 und GM-CSF.

10. Verwendung nach Anspruch 2, wobei sich eine therapeutisch wirksame Menge des Gens an der Neoplasie angesammelt hat.

11. Verwendung nach Anspruch 1, wobei das Nukleinsäure-Lipidpartikel ein protonierbares Lipid mit einer pKa im Bereich von etwa 4 bis etwa 11 umfasst.

12. Verwendung nach Anspruch 11, wobei das protonierbare Lipid ein Mitglied ist, ausgewählt aus der Gruppe, bestehend aus DODAC, DODAP, DODMA, DOTAP, DOTMA, DC-Chol, DMRIE, DSDAC und Gemischen davon.

13. Verwendung nach Anspruch 1, wobei das Nukleinsäure-Lipidpartikel ein Lipidkonjugat umfasst, das die Aggregation während der Formulierung verhindert.

14. Verwendung nach Anspruch 13, wobei das Lipidkonjugat ein Mitglied ist, ausgewählt aus der Gruppe, bestehend aus PEG-Lipiden und PAO-Lipiden.

15. Verwendung nach Anspruch 13, wobei das Lipidkonjugat mit einer äußeren Lipidmonoschicht reversibel verbunden ist, und wobei das Lipidkonjugat aus der äußeren Lipidmonoschicht bei einer schnelleren Rate als PEG-CerC20 herauswechselt.

16. Verwendung nach Anspruch 1, wobei das Nukleinsäure-Lipidpartikel im wesentlichen frei von Detergenzien und organischen Lösungsmitteln ist.

17. Verwendung nach Anspruch 1, wobei sich eine therapeutisch wirksame Menge des Nukleinsäure-Lipidpartikels an der Neoplasie ansammelt.

18. Verwendung nach Anspruch 1, wobei eine therapeutische Wirkung an der Stelle der Neoplasie nachgewiesen wird.

19. Verwendung nach Anspruch 17, wobei die therapeutisch wirksame Menge mehr als etwa 0,5 % einer verabreichten Dosis umfasst.

20. Verwendung nach Anspruch 1, wobei das Nukleinsäure-Lipidpartikel einen Durchmesser von etwa 50 nm bis etwa 200 nm aufweist.

21. Verwendung nach Anspruch 20, wobei das Nukleinsäure-Lipidpartikel einen Durchmesser von etwa 60 nm bis etwa 130 nm aufweist.

22. Verwendung nach Anspruch 20, wobei die Nukleinsäure-Lipidpartikel von gleichmäßiger Größe sind.

23. Verwendung nach Anspruch 1, wobei das Nukleinsäure-Lipidpartikel ein Verhältnis von Nukleinsäure zu Lipid von größer als etwa 3 mg Nukleinsäure pro mmol Lipid aufweist.

24. Verwendung nach Anspruch 23, wobei das Partikel ein Verhältnis von Nukleinsäure zu Lipid von größer als etwa 14 mg Nukleinsäure pro mmol Lipid aufweist.

25. Verwendung nach Anspruch 23, wobei das Partikel ein Verhältnis von Nukleinsäure zu Lipid von größer als etwa 25 mg Nukleinsäure pro mmol Lipid aufweist.

26. Verwendung nach Anspruch 1, wobei die Nukleinsäure zu wenigstens 90 % intakt bleibt, wenn der Partikel, enthaltend etwa 1 µg DNA, mit etwa 100 U DNAse 1 in Verdaupuffer bei 37 °C für 30 min. behandelt wird.

27. Verwendung nach Anspruch 1, wobei das Medikament zur Anwendung mit einem chemotherapeutischen Mittel ist.

28. Verwendung nach Anspruch 1, wobei die Injektion wenigstens einmal pro acht Wochen durchgeführt wird.

29. Verwendung von
a) einem Serum-stabilen Nukleinsäure-Lipidpartikel, das für ein Genprodukt codiert, umfassend eine Nukleinsäure, die vollständig eingekapselt ist innerhalb des Lipids, und
b) einer ersten Verbindung, die durch das Genprodukt zu einer zweiten Verbindung prozessiert wird, wobei eine neoplastische Zelle empfindlicher ist gegenüber der zweiten Verbindung als gegenüber der ersten Verbindung,
in der Herstellung von Medikamenten für die gleichzeitige oder aufeinander folgende Anwendung in der Behandlung einer metastatischen Neoplasie durch Sensibilisieren der neoplastischen Zelle für die erste Verbindung,
wobei das Medikament, umfassend ein Nukleinsäure-Lipidpartikel, systemisch durch intravenöse Injektion an einer Injektionsstelle verabreicht wird, die physisch von der neoplastischen Zelle getrennt ist, und wobei das Partikel die neoplastische Zelle transfiziert.

30. Verwendung nach Anspruch 29, wobei die erste Verbindung in einem Lipid formuliert wird.

31. Verwendung nach Anspruch 29, wobei das Genprodukt ein Mitglied ist, ausgewählt aus der Gruppe, bestehend aus therapeutischen Polypeptiden und therapeutischen Polynukleotiden.

32. Verwendung nach Anspruch 29, wobei das Genprodukt ein Mitglied ist, ausgewählt aus der Gruppe, bestehend aus Suizidenzymen, Toxinen und Ribozymen.

33. Verwendung nach Anspruch 29, wobei das Genprodukt ein Mitglied ist, ausgewählt aus der Gruppe, bestehend aus Herpes simplex-Virus-Thymidinkinase (HSV-TK), Cytosindeaminase, Xanthin-Guanin-Phosphoribosyltransferase, Purinnukleosidphosphorylase, Cytochrom P450 2B1 und Analoga davon.

34. Verwendung nach Anspruch 29, wobei eine therapeutische Wirkung an der Stelle der neoplastischen Zelle nachgewiesen wird.

## Revendications

1. Utilisation d'une particule acide nucléique-lipide stable dans le sérum comprenant une partie acide nucléique qui est totalement encapsulée dans la partie lipidique, dans la fabrication d'un médicament pour le traitement systémique d'une néoplasie métastatique chez un mammifère par injection intraveineuse en un site d'injection qui est physiquement séparé de ladite néoplasie chez ledit mammifère, dans laquelle l'acide nucléique est un acide nucléique thérapeutique.

2. Utilisation selon la revendication 1, dans laquelle ledit acide nucléique comprend un gène expressible.

3. Utilisation selon la revendication 2, dans laquelle ledit gène expressible code pour un membre choisi dans le groupe constitué de polypeptides thérapeutiques et de polynucléotides thérapeutiques.

4. Utilisation selon la revendication 3, dans laquelle ledit gène est un membre choisi dans le groupe constitué de gènes codant pour des enzymes suicide, des toxines et des ribozymes.

5. Utilisation selon la revendication 2, dans laquelle ledit gène code pour un membre choisi dans le groupe constitué de la thymidine kinase du virus de l'herpes simplex (HSV-TK), une cytosine désaminase, une xanthine-guaninephosphoribosyl-transférase, une purine nucléoside phosphorylase, un cytochrome P450 2B1 et des analogues de ceux-ci.

6. Utilisation selon la revendication 2, dans laquelle ledit gène est exogène par rapport au mammifère.

7. Utilisation selon la revendication 2, dans laquelle ledit gène est endogène par rapport au mammifère.

8. Utilisation selon la revendication 2, dans laquelle ledit gène code pour un membre choisi dans le groupe constitué de proto-oncogènes, cytokines, protéines immuno-stimulatrices et protéines anti-angiogènes.

9. Utilisation selon la revendication 2, dans laquelle ledit gène est un membre choisi dans le groupe constitué de IL-2, IL-12, IL-15 et GM-CFS.

10. Utilisation selon la revendication 2, dans laquelle une quantité thérapeutiquement efficace dudit gène est accumulée au niveau de ladite néoplasie.

11. Utilisation selon la revendication 1, dans laquelle ladite particule acide nucléique-lipide comprend un lipide protonable ayant un pKa dans la gamme d'environ 4 à environ 11.

12. Utilisation selon la revendication 11, dans laquelle ledit lipide protonable est un membre choisi dans le groupe constitué de DODAC, DODAP, DODMA, DOTAP, DOTMA, CD-Chol, DMRIE, DSDAC et des mélanges de ceux-ci.

13. Utilisation selon la revendication 1, dans laquelle ladite particule acide nucléique-lipide comprend un conjugué lipidique qui empêche l'agrégation durant la formulation.

14. Utilisation selon la revendication 13, dans laquelle ledit conjugué lipidique est un membre choisi dans le groupe constitué de PEG-lipides et de PAO-lipides.

15. Utilisation selon la revendication 13, dans laquelle ledit conjugué lipidique est réversiblement associé à une monocouche lipidique externe, et dans laquelle ledit conjugué lipidique s'échange à l'extérieur de ladite monocouche lipidique externe à une vitesse plus rapide que PEG-CerC20.

16. Utilisation selon la revendication 1, dans laquelle ladite particule acide nucléique-lipide est sensiblement dépourvue de détergents et de solvants organiques.

17. Utilisation selon la revendication 1, dans laquelle une quantité thérapeutiquement efficace de ladite particule acide nucléique-lipide s'accumule au niveau de ladite néoplasie.

18. Utilisation selon la revendication 1, dans laquelle un effet thérapeutique est détecté au site de ladite néoplasie.

19. Utilisation selon la revendication 17, dans laquelle ladite quantité thérapeutiquement efficace constitue plus d'environ 0,5 % d'une dose administrée.

20. Utilisation selon la revendication 1, dans laquelle ladite particule acide nucléique-lipide a un diamètre d'environ 50 nm à environ 200 nm.

21. Utilisation selon la revendication 20, dans laquelle ladite particule acide nucléique-lipide a un diamètre d'environ 60 nm à environ 130 nm.

22. Utilisation selon la revendication 20, dans laquelle lesdites particules acide nucléique-lipide ont une taille uniforme.

23. Utilisation selon la revendication 1, dans laquelle ladite particule acide nucléique-lipide a un rapport acide nucléique sur lipide supérieur à environ 3 mg d'acide nucléique par mmole de lipide.

24. Utilisation selon la revendication 23, dans laquelle ladite particule a un rapport acide nucléique sur lipide supérieur à environ 14 mg d'acide nucléique par mmole de lipide.

25. Utilisation selon la revendication 23, dans laquelle ladite particule a un rapport acide nucléique sur lipide supérieur à environ 25 mg d'acide nucléique par mmole de lipide.

26. Utilisation selon la revendication 1, dans laquelle ledit acide nucléique reste intact à au moins 90 % lorsque ladite particule contenant environ 1 µg d'ADN est traitée avec environ 100 U de DNAse 1 dans un tampon de digestion à 37°C pendant 30 min.

27. Utilisation selon la revendication 1, dans laquelle ledit médicament est destiné à une utilisation avec un agent chimiothérapeutique.

28. Utilisation selon la revendication 1, dans laquelle ladite injection est réalisée au moins une fois toutes les huit semaines.

29. Utilisation de
a) une particule acide nucléique-lipide stable dans le sérum codant pour un produit de gène comprenant un acide nucléique qui est totalement encapsulé dans le lipide et
b) un premier composé qui est transformé par ledit produit de gène en un second composé, dans laquelle une cellule néoplasique est plus sensible audit second composé qu'audit premier composé, dans la fabrication de médicaments pour une utilisation simultanée ou séquentielle dans le traitement d'une néoplasie métastatique en sensibilisant ladite cellule néoplasique audit premier composé, dans laquelle ledit médicament comprenant une particule acide nucléique-lipide est administré par voie systémique par injection intraveineuse en un site d'injection qui est physiquement séparé de ladite cellule néoplasique, et dans laquelle ladite particule est transfectée dans ladite cellule néoplasique.

30. Utilisation selon la revendication 29 dans laquelle ledit premier composé est formulé dans un lipide.

31. Utilisation selon la revendication 29 dans laquelle ledit produit de gène est un membre choisi dans le groupe constitué de polypeptides thérapeutiques et de polynucléotides thérapeutiques.

32. Utilisation selon la revendication 29 dans laquelle ledit produit de gène est un membre choisi dans le groupe constitué d'enzymes suicide, de toxines et de ribozymes.

33. Utilisation selon la revendication 29 dans laquelle ledit produit de gène est un membre choisi dans le groupe constitué de la thymidine kinase du virus de l'herpes simplex (HSV-TK), une cytosine désaminase, une xanthine-guaninephosphoribosyl-transférase, une purine nucléoside phosphorylase, un cytochrome P450 2B1 et des analogues de ceux-ci.

34. Utilisation selon la revendication 29 dans laquelle un effet thérapeutique est détecté au site de ladite cellule néoplasique.
